# EUROPÄISCHE PATENTANMELDUNG

(11) **EP 3 741 371 A1**
(43) Veröffentlichungstag der Anmeldung: **25.11.2020**
(21) Anmeldenummer: 20156985.2
(22) Anmeldetag: 21.05.2008
(51) Int. Cl.: A61K 31/53, A61K 33/26, A61K 45/06, A61P 7/06, A61P 33/02

(54) **FORMULIERUNGEN ENTHALTEND TRIAZINONE UND EISEN**

(30) Priorität: 01.06.2007 DE 102007025908
(62) Teilanmeldung aus: 17160876.3
(71) Anmelder: Bayer Animal Health GmbH, 51373 Leverkusen (DE)
(72) Erfinder: Hofmann, Stefan, 40764 Langenfeld (DE); Heep, Iris, 50859 Köln (DE); Mundt, Hans-Christian, 40699 Erkrath (DE); Torres Islas, Juan Augustin, San José (CR)
(74) Vertreter: Tier 1 Patents

(57) **Zusammenfassung**

Die vorliegende Erfindung betrifft Formulierungen umfassend Triazinone wie insbesondere Toltrazuril und Diclazuril und poylnukleare Eisen polysaccharid Komplexverbindungen zusammen mit dem Entschäumer Simethicone zur Bekämpfung von Coccidien-Infektionen und Eisenmangelzuständen insbesondere bei Saugferkeln.

## Beschreibung

Die Erfindung betrifft Formulierungen enthaltend Triazinone und Eisen-Verbindungen (Salze und Komplexverbindungen des Eisens), die sich für die gleichzeitige Bekämpfung von Coccidiosen und Eisenmangelzuständen bei Tieren eignen.

Wirtschaftlich erfolgreiche Betriebe zur Fleischerzeugung zeichnen sich in der modernen Zeit durch hochintensive Bewirtschaftung aus, d.h. durch die Haltung einer großen Anzahl von speziell auf die Optimierung des Zuchtzieles ausgewählten Tieren. Kennzeichen sind z.B. ein hoher maschineller Einsatz, Zufütterung von Nahrungsergänzungsmitteln, und ein möglichst geringer Personalaufwand. Im Falle von Ferkelzuchtbetrieben bedeutet dies, dass eine große Anzahl von Sauen, die auf eine hohe Zahl von Ferkeln pro Wurf hin gezüchtet sind, in entsprechend großräumig ausgelegten Stallungen gehalten werden. Die Optimierung des Futters und eine entsprechende Zuchtauswahl ermöglichen ein rasches Wachstum der Ferkel.

Diese Art der Tierhaltung bedingt oft eine Häufung gewisser typischer Erkrankungen und Mangelzustände. Neben Stress, für den insbesondere Schweine in intensiver Haltung sehr anfällig sind, sind dies bei Jungschweinen u.a. Protozoeninfektionen (Coccidiosen) und Eisenmangelzustände, die häufig bereits durch prophlyaktischen Medikamenteneinsatz zurückgedrängt werden müssen.

Coccidiosen stellen bei Tieren häufig auftretende, parasitöse Infektionserkrankungen dar. So verursachen z.B. Protozoen der Gattungen Eimeria, Isospora, Neospora, Sarkosporidien und Toxoplasma weltweit verbreitete Coccidiosen. Wirtschaftlich bedeutsam sind z.B: Infektionen von Schweinen mit Coccidien der Gattung Isospora oder von Rindern mit Coccidien der Gattung Eimeria. Injektionen mit Isospora suis wurden erst in den letzten Jahren als Ursache von Ferkeldurchfällen erkannt und intensiv erforscht. In der Regel erfolgt eine Infektion von der Umwelt auf die Ferkel oder von Ferkel zu Ferkel über Oocysten, die jeweils zwei Sporocysten mit je zwei Sporozoiten enthalten. Die Vermehrung der Parasitenstadien erfolgt in den Epithelzellen der Dünndarmzotten. Zur klinischen Erscheinung der Erkrankung gehört eine nekrotische, entzündliche Zerstörung der Darmepithelzellen mit Zottenatrophie und dadurch Verdauungs- und Resorptionsstörungen. Kennzeichen einer akuten Erkrankung ist ein wässriger, weißlicher bis gelber Durchfall, der zumeist in der 2. bis 3. Lebenswoche auftritt. Infizierte Ferkel haben eine reduzierte Gewichtszunahme. Die Behandlung und Therapie der Erkrankung sind bisher unzureichend gelöst. Antibiotika sind unwirksam, Sulfonamide sind zwar für die Coccidiosebehandlung zugelassen. Ihre Wirkung ist jedoch fraglich und eine häufig zu wiederholende Gabe ist auf jeden Fall ungeeignet für die Praxis. Weitere Behandlungsmöglichkeiten sind widersprüchlich: Durch Verabreichung von z.B. Monensin, Amprolium oder Furazolidon konnte bei experimentell infizierten Ferkeln eine Erkrankung nicht verhindert werden. Bei neueren Untersuchungen konnte in einzelnen Betrieben trotz guter Hygiene bei bis zu 92 % aller Würfe *Isospora suis* identifiziert werden. Diese Art der Erkrankung ist nicht auf Schweine beschränkt sondern tritt auch bei vielen anderen Tierarten auf, z.B. in der Geflügelzucht, bei Kälbern, Lämmern oder in Kleintieren (Kaninchen).

Ein Beispiel für einen Mangelzustand ist ein Defizit an Eisen bei neugeborenen Ferkeln. Bedingt durch schnelles Wachstum in den ersten Tagen nach der Geburt kommt es zur raschen Erschöpfung der körpereigenen Eisenvorräte, die durch externe Quellen ausgeglichen werden müssen. Durch die hohe Zahl an Saugferkeln kann diese Substitution durch Aufnahme der Milch der Muttersau nicht in ausreichendem Maße erfolgen. Findet zudem die Haltung auf Beton- oder Kunststoffböden statt, können die Ferkel auch keine Eisenverbindungen durch Wühlen im Erdboden aufnehmen. Die Ferkel werden anämisch. Ein klinisch signifikanter anämischer Zustand liegt vor, wenn der Hämoglobingehalt des Blutes auf weniger als 80g/L abgesunken ist. Bei der NRC-Empfehlung *(*National Research Council, Nutrient Requirements of Domestic Animals, No. 2, Nutrient Requirements of Swine, National Academy of Sciences, Washington DC, 1973*)* wird 90g/L als ein Mindesthämoglobinwert angegeben, mit dem die Ferkel gesund aufwachsen und keine Anzeichen an Anämie zeigen. Auffällige Symptome wie Gewichtsverlust oder Minderwuchs stellen sich jedoch meist erst ein, wenn der Hämoglobingehalt des Blutes auf Werte unter 80g/L abgesunken ist. Andere Indikatoren für die Eisenversorgung sind Hämokritwert und Zahl der roten Blutkörperchen pro Volumeneinheit. Schwere Eisenmangelanämie führt ebenfalls zum Tod der Jungschweine.

Zur Bekämpfung der genannten Krankheiten und Mangelzustände stehen bereits Präparate zur Verfügung.

Coccidiose kann durch Verabreichung von Wirkstoffen aus der Gruppe der Triazinone erfolgreich bekämpft werden. Hier unterscheidet man zwischen den Triazindionen - Vertreter sind z.B. die Wirkstoffe Clazuril, Diclazuril, Letrazuril - und den Triazintrionen mit den Wirkstoffen Toltrazuril, Toltrazuril-Sulfoxid und Ponazuril. Triazine, inbesondere Toltrazuril, Ponzazuril oder Diclazuril, sowie ihre Wirkung gegen Coccidien sind aus einer Reihe von Veröffentlichungen bekannt, siehe u.a. DE-OS 27 18 799 und DE-OS 24 137 22. Aus WO 99/62519 sind halbfeste wässrige Zubereitungen von Toltrazuril-Sulfon (Ponazuril) bekannt. Bekannt ist auch, dass inbesondere Toltrazuril zur Behandlung der Coccidiose (z. B. Isospora suis) in Schweinen geeignet ist. Siehe auch beispielsweise die folgenden Veröffentlichungen: Don't forget coccidiosis, update on Isosporosis in piglets. Part I, Pig Progress volume 17, No2, 12-14; Mundt., H.-C., A. Daugschies, V. Letkova (2001): be aware of piglet coccidiosis diagnostits. Part II, Pig Progress volume 17, No 4, 18-20; Mundt, H.-C., G.-Pl Martineau, K. Larsen (2001): control of coccidiosis Part III, Pig Progress volume 17, No 6, 18-19.

Coccidiosen bei Rindern durch Infektionen mit verschiedenen pathogenen Eimeria spp. (z.B. E. bovis und E. zürnii) äußern sich als Durchfallerkrankungen unterschiedlichen Schweregrades bis hin zu blutigen Durchfällen mit Mortalität.

In WO 96/38140, DE 10049468, DE 19958388, WO 00/19964, WO 99/62519 oder WO 00/37063 sowie in DE 102006038292.7 werden Mittel gegen Coccidiose bei Tieren beschrieben. Neben anderen Anwendungsarten wird dort in allgemeiner Form auch die orale Anwendung erwähnt.

In DE 19603984 sind Granulate zur oralen Applikation enthalten. In DE 19824483 werden halbfeste, wässrige Zubereitungen (Pasten) für die Behandlung von Tieren beschrieben. EP 0116175 beschreibt oral applizierbare Lösungen.

Im Bereich der Gefügelzucht kommen häufig trinkwasserlösliche Präparate bzw. Trinklösungen zum Einsatz, während die Wirkstoffe in Großtierbetrieben eher dem Futter zugesetzt werden oder als Suspension über einen Applikator (Drench) oral verabreicht werden. Bedeutung am Markt haben hier z.B. Diclazuril (2,6-dichloro-α-(4-chlorophenyl)-4-(4,5-dihydro-3,5-dioxo-1,2,4-triazin-2(3H)-yl)benzeneacetonitril; CAS Nr 101831-37-2) (CLINACOX™ 0,5%, Janssen Animal Health; VECOXAN™, Biokema SA) zur Futterzumischung und Toltrazuril (1-Methyl-3-[3-methyl-4-[4-[(trifluoromethyl)thio]phenoxy]phenyl]-1,3,5-triazine-2,4,6(1H,3H,5H)-trion; CAS Nr 69004-03-1). Toltrazuril ist zum Beispiel als Trinkwasser-Formulierung für Geflügel und als orale Suspensionsformulierung u.a. für die Behandlung von Saugferkeln im Markt verfügbar. Empfohlen wird, dem Ferkel eine Dosis von 20mg/kg Körpergewicht am 3.-5. Tag nach der Geburt zu verabreichen.

Nachteilig ist der relativ hohe Arbeitsaufwand bei der oralen Verabreichung der erwähnten Anticoccidia (manchmal auch nicht ganz zutreffend als Coccidiostatika bezeichnet): Die Ferkel müssen dazu eingefangen werden und bekommen das Produkt mit Hilfe eines Applikators oder einer Drenchpistole in den Schlund verabreicht. Das Verfahren verursacht zudem einen nicht unbeträchtlichen Stress für die Ferkel.

Zur Vorbeugung der Eisenmangelanämie stehen eine Reihe recht unterschiedlicher Eisenpräparate zur Verfügung, die sich sowohl im Verbindungstyp als auch in der Applikationsweise und der Bioverfügbarkeit unterscheiden. Man unterscheidet (I) einfache anorganische Fe(2+)Salze, (IIa) Komplexverbindungen des Fe(2+) mit organischen Liganden, z.B. mit Milchsäure, oder (IIb) des Fe(3+), z.B. mit Zitronensäure, und (III) polymerartige Komplexverbindungen eines Fe(3+)-oxo-hydroxo-komplexes β-FeO(OH) vom Akaganeit-Typ mit Kohlenhydraten/Polysacchariden, speziell mit oligomeren oder polymeren Kohlenhydratverbindungen, wie z.B. mit Dextran oder mit Dextrin/Polymaltose. Im Folgenden werden unter polymeren Kohlenhydraten/ Kohlenhydratverbindungen und unter Polysacchariden sowohl oligomere als auch polymere Verbindungen verstanden.

Üblich und lange bekannt sind oral zu verabreichende Präparate des Typs (I), wie z.B. die Verwendung von Eisensalzen als Futterzusatzstoffe. In diesen Verbindungen liegt das Eisen in Form von Eisen(2+)-Ionen vor, z.B. als Eisensulfat FeSO₄. Diese Produkte können sowohl dem Futter der Zuchtsau zugesetzt als auch den Ferkeln direkt oral eingegeben werden. Meist werden den Ferkeln mehrere Einzeldosen in den ersten Lebenstagen während der Wachstumsperiode verabreicht, um die relativ geringe Bioverfügbarkeit auszugleichen. Ein alternativer Weg zur Vermeidung mehrfacher Applikation ist die spätere Gabe eisenhaltigen Zusatzfutters (Prestarter und Starter-Futter). Während in den anorganischen Eisen(2+)salzen die Eisenionen durch Dissoziation rasch freigesetzt werden, ist die Freigabe in den Eisen(2+)Komplexverbindungen etwas verzögert. Die Resorption freier Fe(2+) Ionen aus Eisensalzen findet im oberen Dünndarm statt. Die Löslichkeit von Fe(2+) ist unter den physiologischen Bedingungen des oberen Dünndarms um viele Zehnerpotenzen größer als die des Fe(3+) (Forth, W., in: Dünndarm, Handbuch der inneren Medizin, Bd. 3 Verd.Org. Teil 3(A) ; W.F.Caspary, Hrsg.; Springer 1983*).* Freie Eisen(3+) Ionen werden darüber hinaus im Milieu des Darminhalts durch Zystein, Glutathion, Ascorbinsäure u.a. Stoffe zu Fe(2+) reduziert und als solche von den Schleimhaut-Epithelzellen des Darms aufgenommen. Ob diese Reduktion eine notwendige Vorraussetzung für die Aufnahme in die Mucosazellen ist, ist jedoch umstritten. Wahrscheinlich ist der Konzentrationsgradient aufgrund der höheren Löslichkeit des Fe(2+) der Grund für dessen höhere Bioverfügbarkeit. Nach heutiger Auffassung erfolgt die Bindung der Fe(2+) Ionen zunächst an das Protein Mobilferrin, an dem sie wieder zu Fe(3+) oxidiert und an das mucosale Speicherprotein Ferritin gebunden werden. Bei Eisenbedarf des Körpers werden diese Fe(3+) an das Blutplasma abgegeben, wo sie durch eine Ferrioxidase wiederum zu Fe(2+) reduziert und and das Protein Apo-Transferrin gebunden werden, dem Eisen-bindenden Transportprotein des Organismus. Umstritten ist, ob sich Transferrine bereits in den Mucosazellen befinden und dort wenigstens teilweise das Eisen in Empfang nehmen. Die Komplexbildungskonstante des Transferrins ist mit log K ca. 30-31 so groß, dass nirgendwo im Organismus freies Eisen existieren kann, solange die Eisenbindungskapazität des Transferrins nicht überschritten wird. Hierin liegt der Grund für toxische Effekte, die bei einem plötzlichen Überangebot der Eisensalze auftreten können, was einen Nachteil bei deren Verwendung bedeutet. Über Blut und Lymphgefäße wird das Eisen dann an die Orte der Hämoglobinsynthese im Knochenmark gebracht (vgl. E.Kolb, U.Hofmann "Anwendungen von Eisenverbindungen beim Schwein" Tierärtzl. Umschau 60, (2005) 365-371 *und* Forth, W. "Eisen und Eisenversorgung des Warmblüterorganismus", Naturwissenschaften 74, (1987) 175-180 *sowie* John, A. ; "Neue Möglichkeiten der Eisenversorgung neugeborener Ferkel unter Beachtung biochemischer Aspekte" in: Trächtigkeit und Geburt beim Schwein, 8.Bernburger Biotechnik-Workshop 2002 , 89-94*).* Nicht benötigtes Eisen bleibt in den Mucosazellen gespeichert, ist aber nach deren Absterben nicht mehr verfügbar. Somit ist verständlich, dass die Bioverfügbarkeit oraler Eisenverbindungen stark von anderen Faktoren wie tatsächlichem Eisenbedarf, Fütterungszustand (Kolostralmilch) und Gesundheitszustand (Diarrhoe: vorzeitiger Verlust der oberen Mucosazellen) abhängt. Das Verständnis des Mechanismus ist wichtig, um Vor- und Nachteile bestimmter Eisenpräparate verstehen und einschätzen zu können.

Desweiteren werden Verbindungen aus der zweiten Gruppe (II) der chelatartig komplexierten Fe(2+) und Fe(3+) Verbindungen verwendet. Diese bilden relativ stabile Eisenkomplexe, die nur teilweise durch die Magensäure in die Ionen aufgebrochen werden. Die Bioverfügbarkeit ist im weiteren Verlauf dadurch bestimmt, dass das Eisen teilweise mit den Liganden an oder in den Mucosazellen ausgetauscht wird, was von den Bildungskonstanten der Komplexe abhängt. Nicht aufgebrochene Komplexe können wegen der höheren Lipophilie die Membransysteme des Epithels durchqueren und müssen metabolisiert werden. Dies erklärt, warum organische niedermolekulare Komplexe langsamer bioverfügbar sind, aber dafür nachhaltigere Wirkung zeigen *(*H. Dietzfelbinger; "Bioavailability of Bi- and Trivalent Oral Iron Preparations"; Arzneim.-Forsch. / Drug. Res 37(1), Nr. 1a, (1987) 107-112 *und* E.B.Kegley et al., "Iron Methionin as a Source of Iron for the Neonatal Pig", Nutrition Research 22 (2002) 1209-1217*).*

Die dritte Gruppe von Verbindungen, die hauptsächlich parenteral und nur in geringerem Umfang oral appliziert werden, besteht aus recht stabilen Verbindungen des Typs Poly-ß-FeO(OH) mit komplexgebundenen polymeren Kohlenhydraten. Hier haben hauptsächlich, aber nicht ausschließlich, Eisen(III)-Dextran (CAS Nr. 9004-66-4), Eisen(III)-hydroxid-polymaltose (Eisen(III)-hydroxid-Dextrin; CAS Nr. 53858-86-9) Eisen(III)-Saccharose (Eisen(III)-Sucrose, Eisen-(III)"Zucker" CAS Nr. 8047-67-4) und Natrium Eisen(III)-Gluconat-Komplex in Saccharose-Lösung (CAS Nr. 34089-81-1) kommerzielle Bedeutung erlangt. In der Literatur finden sich unterschiedliche Benennungen dieser Verbindungen. Unter Verbindungen wie Eisen(III)-Dextran, -Polymaltose, -Dextrin, -Saccharose, -Gluconat, -Zucker werden hier Komplexe des Eisen(3+)-Ions mit Hydroxid-Ionen (OH⁻), Aquo-Gruppen (H₂O) und Sauerstoff (O) verstanden, die in oligo- oder polymerer Form vorliegen, und mit einer oder mehreren der genannten oligomeren und polymeren Kohlenhydratverbindungen in ihrer Koordinationsphäre komplex assoziiert sind. Aus diesem Grund werden die Verbindungen auch als Eisen(III)-hydroxid-Polysaccharid oder Eisen(III)-oxy-hydroxy-Polysaccharid bezeichnet, wobei Polysaccharid für die oben genannten oligo- und polymeren Kohlenhydratverbindungen oder deren Derivate oder allgemein für Verbindungen aus der Gruppe der oligomeren oder polymeren Kohlenhydrate steht. Polynukleare Eisen(III)-Komplexe dieser Art sind zum Beispiel beschrieben in *(*D.S.Kudasheva et al. "Structure of Carbohydrate-bound Polynuclear Oxyhydroxide Nanoparticles in Parenteral Formulation", J. Inorg. Biochem. 98 (2004) 1757-1769*;* I.Erni et al "Chemical Characterization of Iron(III)-Hydroxide-Dextrin Complexes" Arzneim.-Forsch. / Drug Res. 34 (II) (1984) 1555-1559*;* F.Funk et al. "Physical and Chemical Characterization of Therapeutic Iron Containing Materials", Hyperfine Interactions 136 (2001) 73-95*;* E.London "The Molecular Formula and Proposed Structure of the Iron-Dextran Complex, IMFERON", J. Pharm. Sci. 93 (2004) 1838-1846*;* A.John "Neue Möglichkeiten der Eisenversorgung neugeborener Ferkel unter Beachtung biochemischer Aspekte", Trächtigkeit und Geburt beim Schwein: 8.Bernburger Biotechnik-Workshop, Bernburg (2002) 89-94*.).* Da die Zusammensetzung dieser Verbindungen in vielen Fällen nicht quantitativ beschrieben ist und innerhalb der Verbindungen auch je nach Herstellungsart variieren kann, sollen unter diesen polynuklearen Eisen(III)-Polysaccharid Verbindungen alle dem Fachmann geläufigen Komplexe der beschriebenen Verbindungsklasse verstanden sein.

Diese Eisen-Verbindungen finden fast ausschließlich bei der Herstellung von Injektionspräparaten für die Human- und Veterinärmedizin Verwendung. In der Veterinärmedizin sind jedoch auch einige wenige oral zu verabreichende Präparate im Gebrauch. Diese Komplexe zeichnen sich allgemein durch hohe Stabilität aus und unterscheiden sich hauptsächlich in ihrem Molgewicht, das von 30kDa bis hin zu 400kDa variieren kann, sowie in der Stärke der Komplexbindung. In wässriger Lösung liegen sie als kolloidale Dispersionen mit 7-35nm Partikelgröße vor. Ausschlaggebend für die Bioverfügbarkeit im Falle der oralen Anwendung ist zum einen das Ausmaß der Präzipitatbildung und der Hydrolyse des Eisenkerns unter dem Einfluss der Magensäure, zum anderen die Stabilität der Komplexe unter sauren reduzierenden Bedingungen. Die Mechanismen der Aufnahme in den Organismus und des Umbaus zu biologischen Eisenverbindungen sind noch nicht völlig geklärt und in der Literatur zum Teil auch noch umstritten. Es lassen sich aber einige generelle Aussagen zum Wirkungsmechanismus treffen. Je stabiler der Komplex, desto höher ist der Anteil der Verbindung, der die Magenpassage unverändert übersteht und desto geringer ist der Anteil freier Eisenionen. Die Stabilität der Komplexe wiederum hängt vom Syntheseverfahren ab. Hochmolekulare Eisen(III)-Polymaltose und Eisen(III)-Dextran haben sich als recht stabil erwiesen. Demgegenüber steht die Notwendigkeit, das Eisen an die Proteine des Transportweges abzugeben. Dieser Transfer ist naturgemäß umso geringer, je stabiler die Komplexe sind. Diese Zusammenhänge wurden durch verschiedene Versuche mit Säuren, reduzierenden Agentien und mit Komplexbildnern belegt. (R. Lawrence "Development and Comparison of Iron Dextran Products" ; PDA J Pharm. Sci. Techn. 52(5) (1998) 190-197*;* F.Funk et al "Physical and Chemical Characterization of Therapeutic Iron Containing Materials"; Hyperfine Interactions 136 (2001) 73-95*;* I.Erni et al "Chemical Characterization of Iron(III)-Hydroxide-Dextrin Complexes" Arzneim.-Forsch. / Drug Res. 34(II) 11 (1984) 1555-1559*).*

Aus diesen Überlegungen heraus ist die Lehrmeinung entstanden, dass Fe(3+)-Verbindungen im Allgemeinen, und insbesondere polynukleare Verbindungen wie Eisen(III)-Dextran, nicht für orale Applikation geeignet sind *(*H.Dietzfelbinger "Bioavailability of Bi- and Trivalent Oral Iron Preparations" Arzneim.-Forsch. / Drug Res. 37(I), Nr 1(a) (1987) 107-112*.)*

Ein weiterer Grund für die Skepsis gegenüber der oralen Verwendung von polynuklearen Fe(3+)Komplexen, insbesondere Eisen(III)-Dextran, ist der spezielle Aufnahmeweg der ß-FeO(OH)-Komplexe im Intestinaltrakt. Diese Verbindungen werden durch Pinocytose in die Epithelzellen der Darmschleimhaut aufgenommen und müssen dann an den Organismus über die Lymphbahnen abgegeben, in den Lymphknoten gespeichert und schließlich in die Blutbahn weiter transportiert werden (*vgl. auch bereits genannte Artikel von Kolb, Hofmann; Forth; John).* Da sie - wie dargelegt - recht stabil sind, hängt die Bioverfügbarkeit im weiteren Verlauf vom Metabolismus und enzymatischen Abbau der Komplexe mit lysosomalen Enzymen ab. Aus Versuchen mit Polyvinylpyrrolidon, Dextran und mit Farbstoff-markierten Eisen(III)-Dextran jeweils unterschiedlichen Molekulargewichts ist bekannt, dass bei Saugferkeln die Aufnahme dieser Polymerkomplexe durch die Pinocytose über die Epithelzellen des Zwölffingerdarms und des oberen Dünndarms in den ersten Lebenstagen erfolgen kann (R.M.Clarke, R.N. Hardy "Histological Changes in the Small Intestine of the Young Pig and Their Relation to Macromolecular Uptake"; J. Anat. 108(1), (1971) 63-7*;* K.Thoren-Tolling, L.Jönsson "Cellular Distribution of Orally and Intramuscularly Administered Iron Dextran in Newborn Piglets", Can. J. Comp. Med. 41 (1977) 318-325*;* K. Martinsson, L.Jönsson "On the Mechanism of Intestinal Absorption of Macromolecules in Piglets Studied with Dextran Blue", Zbl. Vet. Med. A 22 (1975) 276-282*).* Es ist jedoch ebenfalls bekannt, dass dieser Übergang hochmolekularer Verbindungen von den Mucosazellen in die Lymph- und Blutbahnen der Ferkel nur unmittelbar nach der Geburt ungehindert möglich ist. Dieser Mechanismus garantiert, dass die Versorgung von Ferkeln mit Imunoglobulinen und Antikörpern durch die Aufnahme der Kolostralmilch der Sau sofort nach der Geburt stattfinden kann. Sobald diese Versorgung gewährleistet ist, kommt der Transportmechanismus zum Erliegen. Diese Schleimhautblockade ("Intestinal Closure") im weiteren Verlauf des Wachstums ist biologisch sinnvoll, um Infektionen mit Mikroorganismen und Giftstoffen zu vermeiden *(*K. Martinsson, L.Jönsson "The Uptake of Macromolecules in the Ileum of Piglets after Intestinal Closure", Zbl. Vet. Med. A 23 (1976) 277-282*).* Der Zeitraum zwischen Geburt und Schleimhautblockade ist deshalb stark vom Ernährungszustand der Ferkel abhängig. Bei hungernden Ferkeln kann dieser Transfer noch bis zu vier Tagen nach der Geburt stattfinden *(*J.G. Lecce, D.O. Morgan "Effect of Dietary Regimen on Cessation of Intestinal Absorption of Large Molecules (Closure) in the Neonatal Pig and Lamb", J. Nutrition 78 (1962) 263-268*).* Da die allgemeinen Haltungsbedingungen in den Zuchtbetrieben aber naturgemäß das Saugen ermöglichen, ist gegenwärtig allgemein medizinisch anerkannt und Stand des Wissens, dass eine ausreichende Versorgung von Ferkeln mit hochmolekularen Eisen-Komplexen auf dem oralen Wege nur in den ersten Stunden nach der Geburt sinnvoll möglich ist, wenn man Mehrfachapplikationen vermeiden möchte. Die wenigen Autoren, die systematisch die Wirksamkeit von Eisen(III)-Dextran in Abhängigkeit von Zeitpunkt der oralen Applikation untersucht haben, berichten von einer deutlich verminderten Wirksamkeit, falls das Eisen(III)-Dextran 24-72h nach der Geburt gegeben wird (L.Blomgren, N.Lanneck "Prevention of Anaemia in Piglets by a Single Oral Dose of Iron Dextran", Nord. Vet.-Med. 23 (1971) 529-536*).* Abhängig von Haltungs- und Fütterungsbedingungen kann allerdings auch eine Gabe am 2. Lebenstag noch ausreichend gute Ergebnisse erzielen (S.Kadis, "Relationship of Iron Administration to Susceptibility of Newborn Pigs to Enterotoxic Colibacillosis"; Am. J. Vet. Res. 45(2), (1984) 255-259). Bei einer Gabe des Eisen-Dextrans 72-96h nach der Geburt ist die Effektivität demgegenüber bereits stark reduziert *(*Ueda H. "Prevention of Piglet Anemia by Oral Administration of Iron Dextran", Nicchiku Kaiho 56(11), 1985, 872-877*).* Am Markt haben sich deshalb nur wenige orale Eisensubstitutionspräparate mit polynuklearen Eisen-Komplexen durchgesetzt (*Ursoferran 150 p.o.; Fa Serumwerke Bernburg* - *Eisen(III)-Dextran ; Ferrum Hausmann Syrup*® *Hausmann Laboratories Inc., St.Gallen;* - *Eisen(III)-hydroxid polymaltose.* In modernen Eisen-Dextran Präparaten zur oralen Verwendung bei Zuchtferkeln wird das Eisen-Dextran an die Emulgatoren von 1-2µm großen Mikroemulsionströpfchen gebunden, um die Bioverfügbarkeit zu verbessern *(Bioveyxin FeVit™, Fa. Veyx-Pharma GmbH, Schwarzenborn; SintaFer™, Fa. Sinta GmbH, Schwarzenborn)* Dieser fein dispergierte Zustand und die Anbindung an lipophile Träger soll die Aufnahme in die Epithelzellen und den Transfer in den Organismus fördern. Selbst für diese Präparate wird jedoch eine Anwendung bis zu maximal 8-10h nach der Geburt vom Hersteller empfohlen, um optimale Wirkung zu erzielen. Das wiederum setzt eine kontinuierliche Überwachung der Zuchtsauen voraus, was einen erheblichen personellen Aufwand bedeutet.

Generell wird für orale Eisenpräparate eine Dosierung von 100-200mg aktivem Eisen pro Ferkel und Dosiseinheit empfohlen, um ausreichende Wirksamkeit zu gewährleisten. Nur die höhere Dosis ermöglicht es in der Praxis jedoch, mit einer einmaligen Verabreichung auszukommen.

Um die beschriebenen Unwägbarkeiten bei einer oralen Applikation zu umgehen, ist es in der Schweinezucht üblicher, polynukleare Eisen(III)-Komplexe per Injektion intramuskulär zu verabreichen. Das erfolgt in der Regel durch eine Injektion von 100-200mg aktiven Eisens am 3.Tag nach der Geburt. Der Abtransport vom Injektionsort erfolgt über das lymphatische System und die Zellen des retikulohistiocytären Systems. Die Speicherung der Komplexe findet in Leber und Milz statt, woraus es bedarfsabhängig freigesetzt und enzymatisch metabolisiert wird. Das freie Fe(3+) wird letztlich wieder an Transferrin gebunden und an die Orte der Verwendung im Knochenmark verbracht.

Aber auch diese parenterale Applikationsform weist eine Reihe von Nachteilen auf: Ein deutlicher Nachteil der i.m. (intramuskuläre Injektion) Applikation an Jungschweinen ist, dass schädliche Effekte häufiger auftreten. An den Injektionsstellen werden häufiger Muskelblutungen, Veränderungen der Muskelfasern, Entzündungen und Ödembildungen verursacht. Dies sind lokale Schädigungen. Jedoch werden auch Störungen des Herzmuskels beobachtet, insbesondere bei gleichzeitig vorliegendem Mangel an Vitamin E. Es kann in diesen Fällen eine starke Zunahme des Kalium Gehalts im Blutplasma festgestellt werden, was den Herzmuskel stark schädigt und bis zum Tod der Ferkel führen kann. Nach gegenwärtiger Ansicht sind kleinste Mengen freier Fe(2+) Ionen für eine Bildung radikalischer Verbindungen mit organischen Molekülen verantwortlich, z.B. die Lipid-Peroxidverbindungen, die mit dem hohen Gehalt an Kalium im Blut einhergehen. Vitamin E wirkt als Radikalfänger und kann diese schädlichen Reaktionen in gewisser Weise abpuffern, wobei die körpereigene Kapazität aber häufig überfordert wird. (Den bereits erwähnten oralen Präparaten, bei denen das Eisen(III)-Dextran an Mikroemulsionströpfchen gebundenen ist, wird deshalb noch Vitamin E zugesetzt). Die intramuskuläre Applikation birgt aber noch in anderer Hinsicht einen Nachteil: Nach Verabreichung des Eisen(III)-Dextrans ist mit einer gewissen Einschränkung der Leistungsfähigkeit des Immunsystems zu rechnen, da die Makrophagen im Blut mit den polynuklearen Eisen-Komplexen beladen werden. Die Abwehrfähigkeit bakterieller Infektionen ist vermindert. Eine Übersicht über die beschriebenen Nachteile der i.m. Verabreichung findet sich in der Literatur (E.Kolb, U.Hofmann "Zur Frage der zweckmäßigen Form der Anwendung von Fe-Dextran, seiner Verwertung sowie des Mechanismus einer möglichen Schädigung der Ferkel"; Mh. Vet.-Med. 44 (1989) 497-501*).*

Zusammenfassend kann also festgestellt werden, dass die gegenwärtig am Markt verfügbaren Methoden der Anämieprophylaxe bei Saugferkeln jeweils eine Reihe von Nachteilen aufweisen:
1. Bei oraler Anwendung von Fe(II)-Verbindungen des Typs (I) und (II) ist laut Literatur meist eine deutlich geringere Bioverfügbarkeit zu beobachten. Es wird empfohlen, diese Präparate mehrmals zu verabreichen, was bei der intensiven Tierhaltung personalaufwändig ist und einen wirtschaftlichen Nachteil darstellt.
2. Eine orale Anwendung polynuklearer Fe(III)-Verbindungen des Typs (III), insbesondere Eisen(III)-Dextran, führt zwar zu besseren Ergebnissen. Eine einmalige Dosierung von etwa 200mg aktiven Eisens reicht in der Regel aus, um eine ausreichende Eisenversorgung der Ferkel zu gewährleisten. Ein entscheidender Nachteil ist hier jedoch, dass eine ausreichende Wirksamkeit nach gegenwärtiger Lehrmeinung nur dann zu erzielen ist, wenn den Ferkeln das Eisen(III)-Dextran innerhalb der ersten 8-10 Lebensstunden verabreicht werden kann. Dies wäre nur bei einer durchgängigen Geburtenüberwachung in den Zuchtbetrieben zu gewährleisten, die wegen des großen Personalbedarfs häufig nicht möglich ist. Verpasst man diesen Zeitpunkt, sind größere Verlustraten bei den Ferkeln häufig die Folge.
3. Die intramuskulär zu verabreichenden Eisen-Präparate sind vorteilhafter in der Anwendung, da eine Applikation im Zeitraum von Tag 1 bis Tag 3 nach der Geburt zu sehr guten Ergebnissen führt. Von Nachteil ist jedoch deren Potential zur Schädigung der Ferkel durch toxische Nebenwirkungen und kurzzeitige Schwächung des Immunsystems. Orale Präparate zeigen diesen Nachteil nicht.
4. Berücksichtigt man weiter die häufig notwendige Coccidiose-Behandlung beispielsweise mit Toltrazuril oder ähnlichen Verbindungen, wird deutlich, dass für eine erfolgreiche Ferkelzucht sehr häufig zwei Arbeitsgänge - (1) Einfangen der Ferkel am Tag 1 nach der Geburt und Gabe von z.B. Eisen(III)-Dextran, weiteres Einfangen der Ferkel am Tag 3 und orale Gabe einer handelsüblichen Suspensionsformulierung von Toltrazuril oder (2) Einfangen der Ferkel am Tag 3 und getrennte Gabe der handelsüblichen Toltrazuril Suspension zur oralen Applikation sowie einer Injektionsformulierung von Eisen(III)-Dextran (mit den beschriebenen Nachteilen) - nötig sind.

Es wäre somit von großem Vorteil, stünden Präparate zu Verfügung, die es ohne die beschriebenen Nachteile, d.h. ohne schädliche Nebenwirkungen bei zuverlässiger und hoher Wirksamkeit, möglich machen würden, beide Arbeitsgänge zu vereinen. Ein geeignetes Präparat könnte zum Beispiel eine Formulierung des Wirkstoffs Toltrazuril und des Eisen(III)-Dextrans zur oralen Verabreichung an Ferkeln im Lebenszeitraum des 1.-3. Tages nach der Geburt sein. Präparate, die beide Arbeitsgänge vereinen müssten, aber eine Reihe von Bedingungen erfüllen:
- Ausreichende Wirkstoffmenge: In einer Dosiseinheit muss eine für die pharmakologische Wirksamkeit ausreichende Menge eines Anticoccidiums, üblicherweise 20-70mg, z.B. 30mg, 44mg oder 50mg Toltrazuril, und mindestens 100 mg, besser jedoch mindestens 150 mg, bevorzugt 200-250 mg aktiven Eisens (entsprechend z.B. 400-600 mg eines polynuklearen Eisen(III)-Komplexes) zur Anämieprophylaxe - entsprechend den empfohlenen Dosierungen von 20mg Toltrazuril / kg Körpergewicht und 200mg aktiven Eisens pro Ferkel - enthalten sein. Das entspricht einer Konzentration von 2-7 % m/V des Anticoccidiums und 10-25% m/V an aktivem Eisen in der Formulierung (wobei unter % m/V die Masse der betreffenden Komponente in g pro 100 ml Volumen zu verstehen ist).
- Geringes Dosisvolumen zur oralen Applikation: Beispielsweise ist bei Saugferkeln ein Dosisvolumen von etwa 1ml optimal, da bei deutlich höheren Volumina eine vollständige Aufnahme durch die Ferkel oft nicht sichergestellt ist. Größere Flüssigkeitsmengen laufen oft aus dem Maul heraus oder werden erbrochen.
- Geeignete Konsistenz: Die Viskosität sollte in einem Bereich liegen, der die Verabreichung über Drenchpistolen oder Spritzen ermöglicht, z.B. zwischen 10 und 2500 mPas. Ist die Konsistenz zu flüssig, könnte das Präparat nach Applikation aus dem Maul des Tieres herauslaufen, ist sie zu hoch, ist eine massenhafte Verabreichung mittels Spritzen oder Drenchpistolen für den Anwender zu anstrengend und die Tiere, inbesondere Ferkel, haben Schwierigkeiten beim Schlucken.
- Qualität der Formulierung: Die physikalische und chemische Stabilität und die pharmakologische Wirksamkeit müssen gewährleistet sein. So sollte zum Beispiel sichergestellt sein, dass Eisen-Ionen die chemische Stabilität des Anticoccidiums nicht beeinträchtigen. Ferner sollte sichergestellt sein, dass im Falle einer Suspensionsformulierung eine möglichst feindisperse Verteilung des Wirkstoffs erhalten bleibt, die Koagulation oder gar Verklumpung der dispergierten Wirkstoffpartikel ist nachteilig. Hierdurch könnte z. B. die pharmakologische Wirksamkeit beeinträchtigt werden, da die Lösungsgeschwindigkeit und somit die Freisetzung des Wirkstoffs aus den Partikeln im Darm durch die geringere Oberfläche reduziert ist.
- Wirksamkeit bei Verabreichung in einem größeren Zeitintervall nach der Geburt: Eine ausreichende Wirksamkeit gegen Coccidiose und Anaemie bei Verabreichung im Zeitraum von Tag 1 bis Tag 3 nach der Geburt ist wünschenswert, insbesondere bei Einmalapplikation.
- Ausreichende Anämieprophylaxe bei einmaliger Applikation: Die in dem erwähnten geringen Dosisvolumen des Kombinationspräparats zu verabreichende Menge an Eisen sollte genügen, um den Eisen-Bedarf der Ferkel nach einer Einmalapplikation unter normalen Haltungsbedingungen ausreichend abdecken zu können.

Die Kombination von Triazinonen und Eisenpräparaten in einer geeigneten Formulierung ist bislang noch nicht beschrieben worden.

Die Erfindung betrifft:
1. Mittel enthaltend Triazinone der Formeln (I) oder (II) 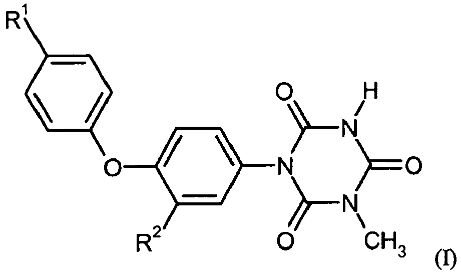 oder 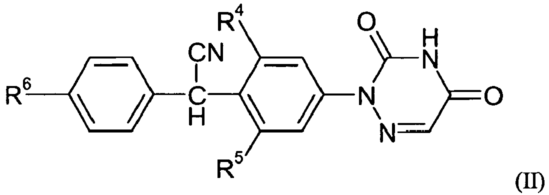 worin
   - R¹: für R³-SO₂- oder R³-S- steht,
   - R²: für Alkyl, Alkoxy, Halogen oder SO₂N(CH₃)₂ steht und
   - R³: für Halogenalkyl steht
   - R⁴ und R⁵: unabhängig voneinander für Wasserstoff oder Cl stehen und
   - R⁶: für Fluor oder Chlor steht.
   oder ihre physiologisch verträglichen Salze
   und
   Eisen(2+) oder Eisen(3+)verbindungen ausgewählt aus:
   (a) Eisen(II)-carbonsäure-salze, Eisen(II)-carbonsäure-komplex-verbindungen und Eisen(II)-chelat-Komplexe mit Aminosäuren
   (b) Eisen(III)-carbonsäure-salzen, Eisen(III)-carbonsäure-komplex-verbindungen und Eisen(III)-chelat-Komplexe mit Aminosäuren und
   (c) polynuklearen Eisen(III)-polysaccharid-Komplexverbindungen.

In den Formeln (I) und (II) haben einzelne Substituenten bevorzugt und besonders bevorzugt folgende Bedeutungen:
- R²: steht bevorzugt für für Alkyl oder Alkoxy mit jeweils 1 bis 4 Kohlenstoffatomen oder für Fluor, Chlor, Brom oder SO₂N(CH₃)₂; R² steht besonders bevorzugt für C₁₋₄-Alkyl.
- R³: steht bevorzugt für Fluoralkyl mit 1 bis 4 Kohlenstoffatomen, besonders bevorzugt für Trifluormethyl.

Die Triazinone sind als Wirkstoffe gegen Coccidien-Infektionen per se gut bekannt, genannt seien die Triazintrione wie z. B. Toltrazuril und Ponazuril sowie die Triazindione wie z. B. Clazuril, Diclazuril und Letrazuril.

Die Triazindione werden durch Formel (II) wiedergegeben:
Clazuril (R⁴ = Cl, R⁵ = H, R⁶ = Cl in Formel (II))
Letrazuril (R⁴ = Cl, R⁵ = Cl, R⁶= F in Formel (II)) und
Diclazuril (R⁴ = Cl, R⁵ = Cl, R⁶= Cl in Formel (II).
Von diesen 1,2,4-Triazindionen ist Diclazuril am meisten bevorzugt.

Erfindungsgemäß besonders bevorzugt sind die Triazintrione der Formel (I) als Wirkstoffe, worin R² und R³ die folgenden bevorzugten und besonders bevorzugten Bedeutungen haben:
- R²: steht bevorzugt für Alkyl oder Alkoxy mit jeweils bis zu 4 Kohlenstoffatomen, besonders bevorzugt für Methyl, Ethyl, n-Propyl, i-Propyl.
- R³: steht bevorzugt für Perfluoralkyl mit 1 bis 3 Kohlenstoffatomen, besonders bevorzugt für Trifluormethyl oder Pentafluorethyl.

Die bevorzugten Triazintrione werden durch die Formel (I) wiedergegeben:
Toltrazuril (R¹ = R³-S-, R² = CH₃, R³ = CF₃)
Ponazuril (R¹ = R³-SO₂-, R²= CH₃, R³ = CF₃)
Die Dosierung des Triazinons kann - wie oben erläutert - je nach Tierspezies variieren. Übliche Dosierungen liegen bei 1 bis 60 mg Wirkstoff pro kg Körpergewicht (mg/kg) des zu behandelnden Tieres pro Tag, bevorzugt 5 bis 40 mg/kg und besonders bevorzugt 10 bis 30 mg/kg.

Toltrazuril wird bei der oralen Anwendung üblicherweise wie folgt dosiert:

| | |
|---|---|
| Schwein: | 20 mg/kg Körpergewicht |
| Rind: | 15 mg/kg Körpergewicht |
| Schaf: | 20 mg/kg Körpergewicht |
| Geflügel: | 15 mg/kg Körpergewicht |

Außer bei Geflügel wird Toltrazuril pro Behandlung nur einmal verabreicht, sodass z. B. bei Schwein, Rind und Schaf die angegebenen Dosierungen sowohl pro Tag als auch pro Behandlung gelten
Geeignete Eisen(2+)- oder Eisen(3+)-Verbindungen sind:
(a) Eisen(2+)-carbonsäure-salze, Eisen(2+)-carbonsäure-komplex-Verbindungen und Eisen(2+)-chelat-Komplexe mit Aminosäuren
(b) Eisen(3+)-carbonsäure-salze, Eisen(3+)-carbonsäure-komplex-verbindungen und Eisen(3+)-chelat-Komplexe mit Aminosäuren
(c) polynukleare Eisen(3+)polysaccharid-Komplexverbindungen

Als Beispiele für Eisenverbindungen des Typs (a) seinen genannt: Eisen(II)-Lactat (FeC₆H₁₀O₆), Eisen(II)-Gluconat (FeC₁₂H₂₂O₁₄), Eisen(II)-Fumarat (FeC₄H₂O₄) und Chelatkomplexe des Eisens mit Aminosäuren wie z.B. Eisen(II)-bis-glycinat (Fe(C₂H₄NO₂)₂), Eisen(II)-Methionat (Fe(C₅H₁₀NO₂S)₂) und deren Hydratverbindungen.

Als Beispiele für Eisenverbindungen des Typs (b) sei genannt: Eisen(III)-citrat (FeC₆H₅O₇), Ammonium-Eisen(III)-citrat und ggf. deren Hydratverbindungen.

Unter Eisenverbindungen der Gruppe c) werden hier Komplexe des Eisen(3+)-Ions mit Hydroxid-Ionen (OH⁻), Aquo-Gruppen (H₂O) und Sauerstoff (O) verstanden, die in oligo- oder polymerer Form vorliegen, und mit einer oder mehreren der genannten oligomeren und polymeren Kohlenhydratverbindungen in ihrer Koordinationsphäre komplex assoziiert sind. Aus diesem Grund werden die Verbindungen auch als Eisen(III)-hydroxid-Polysaccharid oder Eisen(III)-oxy-hydroxy-Polysaccharid bezeichnet, wobei Polysaccharid für die betreffenden oligo- und polymeren Kohlenhydratverbindungen oder deren Derivate steht. Polynukleare Eisen(III)-Komplexe dieser Art sind zum Beispiel beschrieben in *(*D.S.Kudasheva et al. "Structure of Carbohydrate-bound Polynuclear Oxyhydroxide Nanoparticles in Parenteral Formulation", J. Inorg. Biochem. 98 (2004) 1757-1769*;* I.Erni et al "Chemical Characterization of Iron(III)-Hydroxide-Dextrin Complexes" Arzneim.-Forsch. / Drug Res. 34 (II) (1984) 1555-1559*;* F.Funk et al. "Physical and Chemical Characterization of Therapeutic Iron Containing Materials", Hyperfine Interactions 136 (2001) 73-95*;* E.London "The Molecular Formula and Proposed Structure of the Iron-Dextran Complex, IMFERON", J. Pharm. Sci. 93 (2004) 1838-1846*;* A.John "Neue Möglichkeiten der Eisenversorgung neugeborener Ferkel unter Beachtung biochemischer Aspekte", Trächtigkeit und Geburt beim Schwein: 8.Bernburger Biotechnik-Workshop, Bernburg (2002) 89-94*.).* Da die genaue Zusammensetzung dieser Verbindungen in vielen Fällen nicht quantitativ beschrieben ist und innerhalb der Verbindungen auch je nach Herstellungsart variieren kann, sollen unter diesen polynuklearen Eisen(III)-Polysaccharid Verbindungen alle Verbindungen verstanden werden, die der Fachmann dieser Verbindungsklasse zurechnet.

Als Beispiele für Eisenverbindungen des Typs (c) seien genannt: polynukleare Eisen(III)-polysaccharid-Komplexverbindungen, in denen ein polynuklearer β-FeO(OH)-Kernkomplex polymere Kohlenhydratverbindungen an den freien Koordinationsstellen assoziiert enthält, z.B., Eisen(III)-Dextran, Eisen(III)-hydroxy-Polymaltose (Eisen(III)-Dextrin), nichtstöchiometrische Verbindungen von β-FeO(OH) mit Sacchariden und Oligosacchariden "Eisen(III)-Saccharose/Sucrose" "Eisen(III)-'Zucker'".

Von den oben genannten Eisenverbindungen bevorzugt eingesetzt werden diejenigen vom Typ (b) und vom Typ (c), wobei letztere besonders bevorzugt sind. Als besonders bevorzugtes Beispiel sei Eisen(III)-Dextran genannt.

Für Tiere geeignete Zubereitungen der erfindungsgemäßen Formulierungen sind vorzugsweise Lösungen, Suspensionen oder Pasten, Gele. Suspensionen oder Pasten sind bevorzugt.

Lösungen werden hergestellt, indem der Wirkstoff oder die Wirkstoffe in geeigneten Lösungsmitteln oder Lösungsmittelgemischen gelöst wird bzw. werden. Gegebenenfalls werden weitere Hilfsstoffe wie Lösungsvermittler, Antioxidantien, Konservierungsmittel, Verdickungsmittel, Haftmittel, pH-Wert regulierende Substanzen, Lichtschutzmittel oder Farbstoffe zugefügt.
Als Lösungsmittel seien genannt: Physiologisch verträgliche Lösungsmittel wie Wasser, Alkohole, wie z. B. einwertige Alkanole (z. B. Ethanol oder n-Butanol), mehrwertige Alkohole, wie Glykole (z. B. Ethylenglykol, Propylenglykol, Tetraglycol/Glycofurol), Polyethylenglykole, Polypropylenglykole, Glycerin; aromatisch substituierte Alkohole wie Benzylalkohol, Phenylethanol, Phenoxyethanol; Ester wie Essigester, Butylacetat, Benzylbenzoat, Ethyloleat; Ether wie Alkylenglykolalkylether (z. B. Dipropylenglykolmonomethylether, Diethylenglykolmonobutylether); Ketone wie Aceton, Methylethylketon; aromatische und/oder aliphatische Kohlenwasserstoffe, pflanzliche oder synthetische Öle; Glycerinformal, Solketal (2,2-Dimethyl-4-hydroxymethyl-1,3-dioxolan), N-Methylpyrrolidon, 2-Pyrrolidon, N,N-Dimethylacetamid, Glycofurol, Dimethylisosorbit, Lauroglykol, Propylencarbonat, Octyldodecanol, Dimethylformamid, sowie Gemische der genannten Lösungsmittel.
Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.
Antioxidantien sind Sulfite oder Metabisulfite wie Kalium- oder Natriummetabisulfit, Natrium- oder Kaliumdisulfit Ascorbinsäure, Iso-Ascorbinsäure, Ascorbinsäurepalmitat, Gallussäureester, Butylhydroxytoluol, Butylhydroxyanisol oder Tocopherole.
Synergisten dieser Antioxidantien können sein: Aminosäuren (z. B. Alanin, Arginin, Methionin, Cystein), Citronensäure, Weinsäure, Edetinsäure oder deren Salze, Phosphorsäurederivate oder Polyalkohole (Polyethylenglykol).
Konservierungsmittel sind: Benzylalkohol, Benzalkoniumchlorid, Trichlorbutanol, p-Hydroxybenzoesäureester, n-Butanol, Chlorocresol, Cresol, Phenol, Benzoesäure, Citronensäure, Weinsäure oder Sorbinsäure.
Verdickungsmittel sind: anorganische Verdickungsmittel wie Bentonite, kolloidale Kieselsäure, Aluminiumstearate, organische Verdickungsmittel wie Cellulosederivate z.B. Hydroxypropylmethylcellulose 4000, Polyvinylalkohole und deren Copolymere, Xanthan, Acrylate und Methacrylate, Carboximethylcellulose und deren Salze.
Haftmittel sind z.B. Cellulosederivate, Stärkederivate, Polyacrylate, natürliche Polymere wie Alginate, Gelatine.
Haftmittel, die auch eine verdickende Eigenschaft haben, können ebenfalls als Verdickungsmittel eingesetzt werden.
pH-Wert regulierende Substanzen sind pharmazeutisch übliche Säuren oder Basen. Zu den Basen zählen Alkali- oder Erdalkalihydroxide (z. B. NaOH, KOH), basische Salze wie z.B. Ammoniumchlorid, basische Aminosäuren wie z.B. Arginin, Cholin, Meglumin, Ethanolamine oder auch Puffer wie z.B. Tris(hydroxymethyl)aminomethan, Citronensäure- oder Phosphatpuffer. Zu den Säuren gehören z.B. Salzsäure, Essig-, Wein-, Citronen-, Milch-, Bernstein-, Adipin-, Methansulfon-, Octan-, Linolensäure, Gluconolacton sowie saure Aminosäuren wie z.B. die Asparaginsäure.
Lichtschutzmittel sind z.B. Stoffe aus der Klasse der Benzophenone oder Novantisolsäure.
Farbstoffe sind alle zur Anwendung am Tier oder Menschen zugelassenen Farbstoffe, die gelöst oder suspendiert sein können.

Suspensionen werden hergestellt, indem man den Wirkstoff oder die Wirkstoffe in einer Trägerflüssigkeit gegebenenfalls unter Zusatz weiterer Hilfsstoffe wie Netzmittel, Farbstoffe, resorptionsfördernde Stoffe, Verdickungsmittel, Haftmittel, Konservierungsstoffe, Antioxidantien, Lichtschutzmittel oder Entschäumer suspendiert.
Als Trägerflüssigkeiten seien genannt alle homogenen Lösungsmittel und Lösungsmittelgemische.
Als Netzmittel (Dispergiermittel) seien genannt:
   Tenside (beinhaltet Emulgatoren und Netzmittel) wie
      1. anionaktive, wie Na-Laurylsulfat, Fettalkoholethersulfate, Mono/Dialkylpolyglykoletherorthophosphorsäureester-Monoethanolaminsalz, Ligninsulfonate oder Dioctylsulfosuccinat,
      2. kationaktive wie Cetytrimethylammoniumchlorid,
      3. ampholytische wie Di-Na-N-lauryl-β-iminodipropionat oder Lecithin,
      4. nichtionogene, z.B. polyoxyethyliertes Rizinusöl, polyoxyethyliertes Sorbitan-Monooleat, Sorbitan-Monostearat, Ethylalkohol, Glycerinmonostearat, Polyoxyethylenstearat, Alkylphenolpolyglykolether, Pluronic®.
Als Entschäumer kommen bevorzugt solche auf Silikon-Basis in Frage, beispielsweise Dimethicon oder Simethicon.
Als weitere Hilfsstoffe seien die weiter oben angegebenen genannt.

Bevorzugt sind Suspensionen und Pasten, wobei bei den Pasten niedrigviskose bevorzugt sind. Bei den Pasten handelt es sich üblicherweise um Suspensionen mit entsprechend höherer Viskosität. Die Suspensionen und Pasten werden vorzugsweise oral verabreicht.

Die erfindungsgemäßen Formulierungen enthalten den Triazinon-Wirkstoff in einer Konzentration von 0,1 bis 30 % (m/V) entsprechend 1 bis 300 mg/ml, bevorzugt 2 bis 25 % (m/V) entsprechend 20 bis 250 mg/ml, besonders bevorzugt 3 bis 15% (m/V) entsprechend 30 bis 150 mg/ml, insbesondere 3 bis 7% (m/V) entsprechend 30-70mg des Triazinons im 1 ml.

Aufgrund der schlechten Löslichkeit der Triazinone liegen diese in den erfindungsgemäßen Formulierungen oft in feinteiliger Form vor. Dabei weist das dispergierte Triazinon eine Partikelgröße (gemessen mit Laserbeugung, Malvern Mastersizer® 2000) von d(v,90) ≤ 30µm, bevorzugt d(v,90) ≤ 20µm, besonders bevorzugt d(v,90) ≤ 10µm, und ganz besonders bevorzugt d(v,90) kleiner oder gleich 7µm auf.

Im Sinne dieser Erfindung wird unter d(v,90) ein volumenbezogene Partikelgrößenverteilung verstanden, bei der 90% aller Partikel eine Dimension (Durchmesser) kleiner oder gleich diesen Wertes aufweisen. Üblicherweise wird diese Angabe mit d(90) bezeichnet, um jedoch deutlich zu machen, dass es sich um eine volumenbezogene Partikelgrößenverteilung handelt, kann die genauere Bezeichnung d(v,90) gewählt werden. Entsprechend sind die Bezeichnungen d(v,50), d(v,10) usw. zu verstehen. Die hier angegebenen Partikelgrößen wurden mit der Methode der Laserbeugung mit dem Gerät Mastersizer 2000 (Dispergiereinheit Hydro 2000G) der Firma Malvern und dem Auswertemodus der Fraunhoferbeugung bestimmt, da die Brechungindices der Wirkstoffpartikel nicht bekannt sind. Eine geeignete Menge der Probenlösung wird dabei mit 2-3ml eines Dispergiermediums (0,1 % wässrige Dioctylnatriumsulfosuccinat Lösung) unter Rühren vordispergiert. Die Dispersion wird dann unter Rühren (300 UpM) und Umpumpen (900 UpM) in die Dispergiereinheit des Geräts gegeben und vermessen. Die Auswertesoftware gibt die Partikelgröße als d(0.5), d(0.9)-Werte usw. aus.

Diese Eisenverbindungen werden in oralen Formulierungen zur Behandlung von Eisenmangelzuständen in Großtierbetrieben üblicherweise in Konzentrationen von 100mg aktiven Eisens bis 200mg aktiven Eisens pro Dosiseinheit als einmalige oder mehrmalige Darreichung appliziert. In Trinklösungen zur Eisenversorgung in Geflügelmastbetrieben kann die Dosis auch weniger als 100mg aktiven Eisens pro Dosiseinheit betragen.

In den erfindungsgemäßen Formulierungen sind die Eisenverbindungen üblicherweise in Konzentrationen von 10% (m/V) bis 30% (m/V) an aktivem Eisen entsprechend 100 bis 300 mg aktiven Eisens in 1 ml, bevorzugt 11,4 % (m/V) bis 25% m/V entsprechend 114mg bis 250 mg aktiven Eisens in 1 ml, besonders bevorzugt jedoch 20% m/V bis 25% m/V entsprechend 200mg bis 250mg aktiven Eisens in 1 ml der Formulierung enthalten. Mit aktivem Eisen bezeichnet man den Prozentsatz an Eisen, der in Form des Eisenkomplexes in der Formulierung enthalten ist. Die Eisenverbindungen liegen in der Regel in den Formulierungen gelöst oder kolloidal gelöst vor. Feinteilige Eisenverbindungen sind in den erfindungsgemäßen Formulierungen weniger bevorzugt.

Vorzugsweise sind die erfindungsgemäßen Formulierungen "auf Wasserbasis". Das bedeutet sie enthalten in der Regel 10 bis 90 Gew.-%, bevorzugt 20 bis 80 Gew.-%, besonders bevorzugt 30 bis 50 Gew.-% Wasser. Beispielsweise können die Formulierungen wie oben angegeben weitere mit Wasser mischbare Lösungmittel enhalten. Als solche seien zum Beispiel bevorzugt mehrwertige aliphatische Alkohole wie Ethylenglykol, Diethylenglykol, Triethylenglykol, Propylenglykol und Glycerin genannt; von diesen ist Propylenglykol besonders bevorzugt. Solche weiteren mit Wasser mischbaren Lösungsmittel sind üblicherweise in Konzentrationen von 1 bis 45 Gew.-%, bevorzugt 1 bis 20 Gew.-%, besonders bevorzugt 5 bis 10 Gew.-% enthalten. Der Zusatz solcher mehrwertiger aliphatischen Alkohole hat auch den Vorteil, dass der Gefrierpunkt der Formulierung gesenkt wird.

Die pro Anwendung zu applizierende Menge an Formulierung hängt davon ab, wieviel Triazinon und Eisen jeweils verabreicht werden soll. Angestrebt werden relativ kleine, leicht per os applizierbare Volumen die je nach Tierart variieren; zum Beispiel werden für Saugferkel Applikationsvolumina von 0,3 bis 2 ml, bevorzugt 0,5 bis 1 ml angestrebt.

Vorteilhaft ist es, wenn die erfindungsgemäßen Formulierungen eine leichte Applikation z. B. mit den üblichen Hilfsmitteln, wie beispielsweise einer Spritze, einem Applikator oder einer Drenchpistole, erlauben und dazu eine flüssige, leicht verdickte oder leicht pastöse Konsistenz aufweisen, die sich daran manifestiert, dass die Viskosität - gemessen durch Mittelwertbildung aus den bei Scherraten 128 s⁻¹ und 256 s⁻¹ mit einer Kegel/Platte Anordnung eines Rheometers (Thermo Scientific RheoStress 600; Kegeldurchmesser 35°; Kegelwinkel 4°; Constant Rate Modus) bei 20°C gemessenen Viskositätswerten - in einem Bereich von 10 bis 2500 mPas, bevorzugt in einem Bereich von 20 bis 1500 mPas, besonders bevorzugt in einem Bereich zwischen 50 und 500mPas und ganz besonders bevorzugt zwischen 20 und 250 mPas liegt. Zur Einstellung eines geeigneten Viskositätsbereichs enthalten die erfindungsgemäßen Formulierungen gegebenenfalls geeignete Substanzen (Verdicker), wie sie bereits weiter oben benannt wurden.

Üblicherweise haben die erfindungsgemäßen Formulierungen einen pH-Wert von 3 bis 8, bevorzugt 4 bis 7, besonders bevorzugt 4 bis 6. Beispiele für geeignete Substanzen zur Regulierung des pH-Wertes wurden bereits weiter oben angegeben. Zum Einstellen des pH-Wertes werden bevorzugt organische Säuren wie z.B. Zitronensäure oder Weinsäure, Mineralsäuren wie z.B. Salzsäure - bevorzugt verdünnte Salzsäure z.B. 0,1 n HCl oder Basen wie z.B. Natronlauge (z.B. 1N-NaOH) eingesetzt.

Weiterhin können die erfindungsgemäßen Formulierungen wie oben angegeben Konservierungsmittel gegebenenfalls in Kombination mit sogenannten Synergisten enthalten. Die Konservierungsmittel sind üblicherweise in Konzentrationen von 0,01 - 5 Gew.-% und speziell mit 0,05 -1 Gew.-% enthalten.

Als Antioxidantien können im Bedarfsfall in den genannten Formulierungen bevorzugt BHA oder BHT eingesetzt werden. Die Konservierungsmittel können für eine ausreichende Konservierung einzeln oder in Kombination auch mit sogenannten Synergisten eingesetzt werden. Synergisten wie Zitronensäure, Weinsäure, Ascorbinsäure oder das Natriumsalz der Editinsäure sind üblicherweise in Konzentrationen von 0,01 - 1 Gew.-%, speziell mit 0,05 - 0,15 Gew.-% enthalten.

Die erfindungsgemässen Formulierungen können gegebenenfalls übliche Entschäumer in Konzentrationen von 0,01 bis 1 Gew.-% enthalten.

Bevorzugt werden die erfindungsgemäßen Formulierungen hergestellt, indem man das Lösungsmittel, vorzugsweise Wasser, vorlegt und darin gegebenenfalls Hilfs- und/oder Zusatzstoffe wie z. B. Colösemittel, Konservierungsmittel, Antioxidantien und viskositätsregulierende Zusatzstoffe vorlöst oder dispergiert. Im bevorzugten Verfahren wird in einem zweiten Schritt das Triazinon, optional in Form eines vorgefertigten Dispersionskonzentrats, unter Verwendung einer leistungsfähigen Homogenisiereinrichtung in diese Vorlösung eingebracht und so lange homogenisiert bis die feinteilige Suspension erreicht ist. Dann wird die Eisen-Verbindung, vorzugsweise in Pulverform, in diese Dispersion eingebracht, wobei erneut homogenisiert wird. Im letzten Schritt schließlich wird der gewünschte pH-Wert durch Zugabe geeigneter pH-Wertregulierender Substanzen eingestellt. Einzelne oder alle Hilfs- und/oder Zusatzstoffe können gegebenenfalls auch nach dem letzten Homogenisierungsschritt zugegeben werden, dies kann z. B. bei bestimmten Verdickern empfehlenswert sein, deren Struktur durch die Homogenisierung zerstört wird.

Die erfindungsgemäßen Formulierungen eignen sich zur kombinierten Bekämpfung von Coccidien und Eisenmangelzuständen, insbesondere bei Tieren. Mit den Formulierungen können die gegen Coccidien wirksamen Triazinone und das Eisen den Tieren in einfacher Weise gleichzeitig zugeführt werden. Die Formulierungen können in der Tierhaltung und Tierzucht bei Nutz-, Zucht-, Zoo-, Labor-, Versuchs- und Hobbytieren vorkommen. Das Wirkspektrum der Triazinone grundsätzlich gut bekannt. Als Coccidien seien hier im Einzelnen genannt:
Mastigophora (Flagellata) wie z.B. Trypanosomatidae z.B. Trypanosoma brucei, T.. gambiense, T. rhodesiense, T. congolense, T. cruzi, T. evansi, T. equinum, T. lewisi, T. percae, T. simiae, T. vivax, Leishmania brasiliensis, L. donovani, L. tropica, wie z.B. Trichomonadidae z.B. Giardia lamblia, G. canis.

Sarcomastigophora (Rhizopoda) wie Entamoebidae z.B. Entamoeba histolytica, Hartmanellidae z.B. Acanthamoeba sp., Hartmanella sp.

Apicomplexa (Sporozoa) wie Eimeridae z.B. Eimeria acervulina, E. adenoides, E. alabahmensis, E. anatis, E. anseris, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Neospora caninum, N. hugesi, Cystisospora spec., Cryptosporidium spec. wie Toxoplasmadidae z.B. Toxoplasma gondii, wie Sarcocystidae z.B. Sarcocystis bovicanis, S. bovihominis, S. neurona, S. ovicanis, S. ovifelis, S. spec., S. suihominis wie Leucozoidae z.B. Leucozytozoon simondi, wie Plasmodiidae z.B. Plasmodium berghei, P. falciparum, P. malariae, P. ovale, P. vivax, P. spec., wie Piroplasmea z.B. Babesia argentina, B. bovis, B. canis, B. spec., Theileria parva, Theileria spec., wie Adeleina z.B. Hepatozoon canis, H. spec.

Ferner Myxospora und Microspora z.B. Glugea spec. Nosema spec.

Ferner Pneumocystis carinii, sowie Ciliophora (Ciliata) wie z.B. Balantidium coli, Ichthiophthirius spec., Trichodina spec., Epistylis spec.

Ganz besonders hervorzuheben sind diejenigen Protozoen Gattungen und Arten, die beim Schwein zu subklinischen oder klinischen Infektionen führen, insbesondere: Eimeria debliecki, E. suis, E. scabra, E. perminuta, E. spinosa, E. polita, E. porci, E. neodebliecki, Isospora suis, Cryptosporidium, Toxoplasma gondii, Sarcocystis miescheriana, S. suihominis, Babesia trautmanni, B. perroncitoi, Balantidium coli.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Tauben, Strauße, Vogelarten für Heim- und Zoohaltung. Ferner gehören dazu Nutz- und Zierfische. Besonders hervorgehoben seien dabei Schweine, Rinder, Schafe und Hunde in allen Arten, Unterarten und Rassen.

Zu Labor- und Versuchstieren gehören Mäuse, Ratten, Meerschweinchen, Goldhamster, Hunde und Katzen.

Zu den Hobbytieren gehören Hunde und Katzen.

Besonders bevorzugt ist die Anwendung bei Schweinen.

Die Anwendung der erfindungsgemäßen Formulierungen erfolgt bevorzugt bei Jungtieren, insbesondere kurz nach der Geburt, bevorzugt bei Saugferkeln. Üblicherweise werden die erfindungsgemäßen Formulierungen (Kombinationspräparat Eisen/Triazinon) nur einmal appliziert. Besonders bevorzugte erfindungsgemäße Formulierungen erlauben eine orale Behandlung von Ferkeln dergestalt, dass eine ausreichende Versorgung der Ferkel mit Eisen in den ersten vier Lebenswochen mit einer einzigen oralen Gabe von 0,7ml - 1,3 ml, bevorzugt 0,7-1,0ml der Formulierung, auch noch am dritten Tag nach der Geburt erreicht werden kann, wobei als Indikator für die ausreichende Versorgung ein Hämoglobinwert von mindestens 8g/100ml Blut, vorzugsweise von mehr als 9g/100ml Blut angesehen werden kann. Zusätzlich soll durch den Triazinonanteil eine wirksame Bekämpfung der Coccidien erreicht werden.

Die erfindungsgemäßen Formulierungen können weitere Wirkstoffe oder Komponenten - allein oder in geeigneten Kombinationen - enthalten, wie z. B. Aufbaustoffe, zu denen beispielsweise Vitamine, Mineralstoffe und als Stoffwechsel- und Immunstimulatien geeignete Phosphorverbindungen zählen:
Vitamine, wie zum Beispiel Vitamin E, Vitamine der B-Reihe wie zum Beispiel Vitamin B12, Vitamin C.

Mineralstoffe, bevorzugt Calcium- oder Magnesiumsalze, insbesondere zum Beispiel Calcium-Gluconat, Calcium-Glucoheptanoat oder Calcium-Sacharat.

Phosphorverbindungen, insbesondere pharmakologisch verträgliche organische Phosphonsäurederivate, die sich als Stoffwechselstimulantien und Tonika eignen. Als bevorzugte Beispiele seien die bereits lange bekannten Verbindungen Toldimfos und insbesondere Butaphosphan genannt.

Gegenstand und bevorzugte Ausführungsformen der Erfindung:
1. Formulierung enthaltend Triazinone der Formeln (I) oder (II) 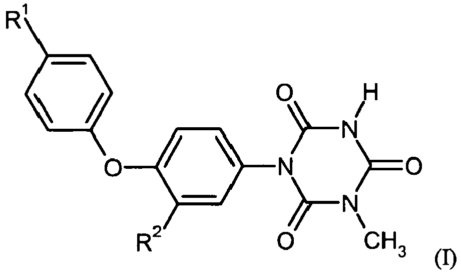 oder 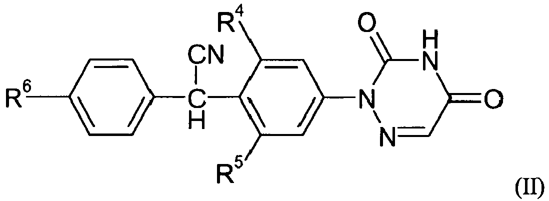 worin
   - R¹: für R³-SO₂- oder R³-S- steht,
   - R²: für Alkyl, Alkoxy, Halogen oder SO₂N(CH₃)₂ steht und
   - R³: für Halogenalkyl steht
   - R⁴ und R⁵: unabhängig voneinander für Wasserstoff oder Cl stehen und
   - R⁶: für Fluor oder Chlor steht.
   oder ihre physiologisch verträglichen Salze
   und
   Eisen(2+) oder Eisen(3+)verbindungen ausgewählt aus:
   (a) Eisen(II)-carbonsäure-salze, Eisen(II)-carbonsäure-komplex-verbindungen und Eisen(II)-chelat-Komplexe mit Aminosäuren
   (b) Eisen(III)-carbonsäure-salzen, Eisen(III)-carbonsäure-komplex-verbindungen und Eisen(III)-chelat-Komplexe mit Aminosäuren und
   (c) polynuklearen Eisen(III)-polysaccharid-Komplexverbindungen
2. Formulierung gemäß Punkt 1 enthaltend 1 bis 30% (m/V), bevorzugt 3-7 % (m/V) an Triazinon.
3. Formulierung gemäß einem der vorstehenden Punkte, wobei das dispergierte Triazinon eine Partikelgröße von d(v,90) kleiner oder gleich 30µm, bevorzugt d(v,90) kleiner oder gleich 20µm, und besonders bevorzugt d(v,90) kleiner oder gleich 10µm aufweist.
4. Formulierung gemäß einem der vorstehenden Punkte mit Konzentration an Eisenverbindung von 10% (m/V) bis 30% (m/V) an aktivem Eisen, bevorzugt 11,4 % (m/V) bis 25% (m/V), besonders bevorzugt jedoch 20% m/V bis 25% (m/V).
5. Formulierung gemäß einem der vorstehenden Punkte mit einer Viskosität - gemessen durch Mittelwertbildung aus den bei Scherraten 128 s⁻¹ und 256 s⁻¹ mit einer Kegel/Platte Anordnung eines Rheometers gemessenen Werten - in einem Bereich von 10 bis 2500 mPas, bevorzugt in einem Bereich von 20 bis 1500 mPas liegt.
6. Formulierung gemäß Punkte 1 auf Wasserbasis .
7. Formulierung gemäß Punkt 1, enthaltend mindestens einen mehrwertigen aliphatischen Alkohol.
8. Formulierung gemäß einem der vorstehenden Punkte enthaltend eine polynukleare Eisen(III)-polysaccharid-Komplexverbindung der Gruppe (c), deren polynuklearer Eisenkern aus β-FeO(OH)-Einheiten besteht und welche Polysaccharidmoleküle in der weiteren Koordinationssphäre enthält.
9. Formulierung gemäß Punkt 8, enthaltend eine polynukleare Eisen(III)-polysaccharid-Komplexverbindung ausgewählt aus: Eisen(III)-Dextran, Eisen(III)-hydroxy-polymaltose / Eisen(III)-Dextrin und einer nicht-stöchiometrischen Verbindung aus polynuklearem ß-FeO(OH) und Saccharose und Oligosacchariden.
10. Formulierung gemäß einem der Punkte 1 bis 7, enthaltend als Eisenverbindung der Gruppe (b) eine Eisen-citrat-Verbindung, bevorzugt Ammonium-Eisen(III)-citrat
11. Formulierung gemäß einem der vorstehenden Punkte enthaltend als Triazinon ein Triazintrion.
12. Formulierung gemäß Punkt 11 enthaltend als Triazintrion Toltrazuril, Ponazuril oder Toltrazuril-Sulfoxid.
13. Formulierung gemäß einem der Punkte 1 bis 8 und 11 bis 12, worin das Triazinon Toltrazuril und die polynukleare Eisen(III)-polysaccharid-Komplexverbindung Eisen(III)-Dextran ist.
14. Formulierung gemäß einem der Punkte 1 bis 10, enthaltend als Triazinon ein Triazindion, insbesondere Clazuril, Diclazuril oder Letrazuril.
15. Verwendung der Formulierungen gemäß einem der vorstehenden Punkte zur Herstellung von Arzneimitteln.
16. Verwendung gemäß Punkt 15 zur Herstellung von Arzneimitteln zur gleichzeitigen Behandlung von Coccidien-Infektionen und Eisenmangelzuständen.
17. Verwendung gemäß Punkt 15 oder 16 zur Herstellung von Arzneimitteln zur oralen Behandlung.
18. Verwendung gemäß Punkt 17 zur Herstellung von Arzneimitteln zur oralen Behandlung von Saugferkeln.
19. Verwendung gemäß einem der Punkte 17 oder 18 zur Herstellung von Arzneimitteln zur oralen Behandlung von Ferkeln in dem Zeitraum von der Geburt bis 10 Tage nach der Geburt, bevorzugt in einem Zeitraum von der Geburt bis 3 Tage nach der Geburt.
20. Formulierungen gemäß einem der Punkte 1 bis 14, zusätzlich enthaltend einen oder mehrere Aufbaustoffe.
21. Formulierung gemäß Punkt 20 zusätzlich enthaltend ein Calcium- oder Magnesiumsalz
22. Formulierung gemäß Punkt 20 oder 21 enhaltend Butaphosphan.

Die nachfolgenden Beispiele sollen die Erfindung erläutern, ohne sie jedoch einzuschränken:

### Herstellbeispiele:

### Beispiel 1

### Verwendung von Eisen(III)-Dextran Pulver 38,4%ig m/m

Ansatz zur Herstellung von 10L einer Eisen-Dextran / Toltrazuril Dispersion (22,8% m/V aktives Eisen + 5% m/V Toltrazuril) zur oralen Anwendung beim Saugferkel

| **Einsatzstoff** | **Masse / g** |
|---|---|
| Toltrazuril Suspensionskonzentrat (30%) | 1666,67 |
| Natrium Propionat (Konservierungsmittel) | 17,00 |
| Natrium Benzoat (Konservierungsmittel) | 17,00 |
| Propylenglycol | 1000,00 |
| Wasserfreie Zitronensäure | 87,10 |
| Eisen(III)-Dextran Pulver 38,4% m/m | 5937,50 |
| Wasser ad 10 Liter | 5138,00 |
| Viskositätsregulierende/r Hilfsstoff/e | - |
| Gesamtmasse | 13863,27 |

Je 17,00 g der Konservierungsmittel Natrium Propionat und Natrium Benzoat werden in einem separaten Gefäß in 1000,00 g des Lösemittels Propylenglycol eingewogen und unter Rühren gelöst. Der gesamte Wasseranteil wird in ein Edelstahlgefäß vorgelegt (Koruma Disho; Maschinen-Type: DH V 100/45). Je nach Bedarf und gewünschter Viskosität des Endprodukts kann in diese Wassermenge ein viskositätsregulierender Hilfsstoff (ein sogenannter Verdicker) gelöst bzw. eingearbeitet werden. Im diesem Beispiel 1 wird darauf verzichtet. Die Propylenglycol-Vormischung wird zu der Vorlage in das Edelstahlgefäß gegeben und unter Rühren (20-40 min) homogenisiert. Im nächsten Schritt wird die vorher abgewogene Menge von 1666,67g des Toltrazuril-Dispersionkonzentrats 30% zu dem Ansatz gegeben, 30-40 min gerührt und gleichzeitig mit einem Umlaufhomogenisator (Rotor/Stator-System) bei 2500 U/min für 20 Minuten homogenisiert. Die genannten Rühr- und Homogenisierzeiten können auch verlängert oder verkürzt werden, je nach Aussehen der Suspension. Es ist vorteilhaft, durch geeignete Kühlsysteme die Temperatur der Mischung auf 20-30°C zu halten. Im nächsten Schritt werden 6015,83g des Eisen(III)-Dextran Pulvers in mehreren Portionen zu der Dispersion gegeben. Während der Zugabe muss ständig gerührt und mit dem Umlaufhomogenisator bei 2500 U/min homogenisiert werden. Die Temperatur wird dabei durch Aktivierung der Kühlung auf 20-30°C gehalten. Nach vollständiger Zugabe des Eisen(III)-Dextran Pulvers wird die Zitronensäure (85,90 g) zu der Mischung gegeben und gelöst. Der pH stellt sich auf 4,1 - 4,4 ein. Nach Einarbeitung aller Komponenten wird nochmals 20 Minuten gerührt und gleichzeitig mit 2500 U/min nachhomogenisiert. Während dieser Nachrührphase wird die Temperatur der Dispersion durch Kühlung auf Raumtemperatur gehalten. Die fertige Dispersion wird über ein 0,1mm Maschensieb aus dem Edelstahlgefäß in geeignete Vorratsbehälter überführt. Der pH-Wert steigt nach einiger Lagerzeit auf Werte zwischen 4,8 und 5,2 an.

Zur Bestimmung der Qualität der Dispersion werden die Werte pH, Teilchengrößenverteilung (gemessen mit Laserbeugung am Malvern Mastersizer 2000) und Viskosität herangezogen. Die Viskosität wird mit einer Kegel/Platte Messanordnung bei einer Scherrate von 128 und 256 s⁻¹ gemessen (RheoStress 600; Thermo Haake). Der Mittelwert der gemessenen Viskositätswerte wird als Referenzwert herangezogen. Diese Viskositätsdaten haben sich als geeignet erwiesen, um den Fließwiderstand einer solchen Suspension bei der Entleerung über eine Drenchpistole zu charakterisieren. Dabei ist grundsätzlich eine möglichst fein verteilte Dispersion anzustreben, um die Bioverfügbarkeit der Aktivstoffe groß zu halten. Die wie beschrieben hergestellte Suspension ergibt dabei folgende Werte:

| Eisen-Verbindung | Viskosität / mPas | pH nach Herstellung | pH nach Lagerung | Partikelgröße d(v,50) / µm | Partikelgröße d(v,90) / µm | Prozentsatz Partikel < 10µm | Prozentsatz Partikel < 30µm |
|---|---|---|---|---|---|---|---|
| Eisen(III)-Dextran Pulver 38,4% m/m | 133 | 4,4 | 5,1 | 2,3 | 4,1 | 100% | 100 % |

### Beispiel 2

### Verwendung von Eisen(III)-Dextran Pulver 36,8%ig m/m

Ansatz zur Herstellung von 1000 ml einer Eisen-Dextran / Toltrazuril Dispersion (23,6% m/V aktives Eisen + 5,3% m/V Toltrazuril) zur oralen Anwendung beim Saugferkel

| **Einsatzstoff** | **Masse / g** |
|---|---|
| Toltrazuril Suspensionskonzentrat (30%) | 177,34 |
| Natrium Propionat (Konservierungsmittel) | 1,89 |
| Natrium Benzoat (Konservierungsmittel) | 1,89 |
| Propylenglycol | 106,38 |
| Wasserfreie Zitronensäure | 7,59 |
| Eisen(III)-Dextran Pulver 36,8% m/m | 639,98 |
| Wasser | 464,67 |
| Viskositätsregulierende/r Hilfsstoff/e | - |
| Gesamtmasse | 1399,74 |

Je 1,89 g der Konservierungsmittel Natrium Propionat und Natrium Benzoat werden in einem separaten Gefäß in 106,38 g des Lösemittels Propylenglycol eingewogen und unter Rühren gelöst. Der gesamte Wasseranteil wird in einem Gefäß (1 L Becherglas) vorgelegt. Je nach Bedarf und gewünschter Viskosität des Endprodukts kann in diese Wassermenge ein viskositätsregulierender Hilfsstoff (ein sogenannter Verdicker) gelöst bzw. eingearbeitet werden. Im diesem Beispiel 2 wird darauf verzichtet. Die Propylenglycol-Vormischung wird zu der Vorlage in das Becherglas gegeben und unter Rühren (10 min) homogenisiert. Im nächsten Schritt wird die vorher abgewogene Menge von 177,34g des Toltrazuril-Dispersionkonzentrats 30% zu dem Ansatz gegeben, und für 30-40 min mit Hilfe einer Dissolverscheibe gerührt. Die genannten Rührzeiten können auch verlängert oder verkürzt werden, je nach Aussehen der Suspension. Im nächsten Schritt werden 639,98g des Eisen(III)-Dextran Pulvers in mehreren Portionen unter Rühren zu der Dispersion gegeben und nach vollständiger Zugabe wird die Suspension für weitere 20 Minuten mit Hilfe der Dissolverscheibe gerührt. Nach vollständiger Zugabe des Eisen(III)-Dextran Pulvers wird die Zitronensäure (7,59 g) zu der Mischung gegeben und gelöst. Der pH stellt sich auf 4,1-4,4 ein. Der pH-Wert steigt nach einiger Lagerzeit auf Werte zwischen 4,8 und 5,2 an.

| Eisen-Verbindung | Viskosität / mPas | pH nach Herstellung | pH nach Lagerung | Partikelgröße d(v,50) / µm | Partikelgröße d(v,90) / µm | Prozentsatz Partikel < 10µm | Prozentsatz Partikel < 30µm |
|---|---|---|---|---|---|---|---|
| Eisen(III)-Dextran Pulver 36,8% m/m | 277 | 4,4 | 4,9 | 3,2 | 6,4 | 99 % | 100 % |

### Beispiel 3

### Verwendung von Eisen(III)-Dextran Lösung 27,5%ig m/V

Ansatz zur Herstellung von 10L einer Eisen-Dextran / Toltrazuril Dispersion (21,0% m/V aktives Eisen + 5% m/V Toltrazuril) zur oralen Anwendung beim Saugferkel

| **Einsatzstoff** | **Masse** / **g** |
|---|---|
| Toltrazuril Suspensionskonzentrat (30%) | 1666,67 |
| Natrium Propionat (Konservierungsmittel) | 17,00 |
| Natrium Benzoat (Konservierungsmittel) | 17,00 |
| Propylenglycol | 1000,00 |
| Wasserfreie Zitronensäure | 150,35 |
| Eisen(III)-Dextran Lösung 27,5% m/V | 11229,00 |
| Viskositätsregulierende/r Hilfsstoff/e | - |
| Gesamtmasse | 14080,02 |

Je 17,00 g der Konservierungsmittel Natrium Propionat und Natrium Benzoat werden in einem separaten Gefäß in 1000,00 g des Lösemittels Propylenglycol eingewogen und unter Rühren gelöst. Die gesamte Menge von 11229,00 g der Eisen(III)-Dextran Lösung wird in ein Edelstahlgefäß vorgelegt (Koruma Disho; Maschinen-Type: DH V 100/45). Je nach Bedarf und gewünschter Viskosität des Endprodukts kann in diese Vorlage ein viskositätsregulierender Hilfsstoff (ein sogenannter Verdicker) gelöst bzw. eingearbeitet werden. Im diesem Beispiel 2 wird darauf verzichtet. Die Propylenglycol-Vormischung wird zu der Vorlage in das Edelstahlgefäß gegeben und unter Rühren (20-40 min) homogenisiert. Die genannten Rührzeiten können auch verkürzt oder verlängert werden, je nach Aussehen der Suspension. Im nächsten Schritt wird die vorher abgewogene Menge von 1666,67g des Toltrazuril-Dispersionkonzentrats 30% zu dem Ansatz gegeben, 30-40min gerührt und gleichzeitig für 20min mit einem Umlaufhomogenisator (Rotor/Stator-System) bei 2500 U/min homogenisiert. Es ist vorteilhaft, durch geeignete Kühlsysteme die Temperatur der Mischung auf 20-30°C zu halten. Im nächsten Schritt wird die Zitronensäure (150,35 g) zugegeben und gelöst. Während der Zugabe ist der Homogenisator mit einer Drehzahl von 1800 U/min eingeschaltet, ebenso wird die Temperatur dabei durch Aktivierung der Kühlung auf Raumtemperatur gehalten. Der pH stellt sich auf 4,1 - 4,4 ein. Die Rühr- und Homogenisierzeiten sind Durchschnittswerte und können je nach Aussehen der Suspension verkürzt oder verlängert werden. Die Kühlung bleibt dabei aktiviert. Die fertige Dispersion wird über ein 0,1mm Maschensieb aus dem Edelstahlgefäß in geeignete Vorratsbehälter überführt. Der pH-Wert equilibriert sich innerhalb von einigen Tagen bis Wochen auf 4,8-5,2.

| Eisen-Verbindung | Viskosität /mPas | pH nach Herstellung | pH nach Lagerung | Partikelgröße d(v,50) / µm | Partikelgröße d(v,90) / µm | Prozentsatz Partikel < 10µm | Prozentsatz Partikel < 30µm |
|---|---|---|---|---|---|---|---|
| Eisen(III)-Dextran Lösung 27,5% m/V | 96 | 4,4 | 4,8 | 2,5 | 4,7 | 100 % | 100 % |

### Beispiel 4

### Verwendung von Eisen(III)-Zucker Pulver 35,9%ig m/m

Verwendet wurde die als Eisen(III)-Zucker (Fa. Dr. Paul Lohmann GmbH KG) bezeichnete Verbindung, wobei es sich um einen Eisen(III)-(hydroxid)-saccharat-Komplex handelt.

Ansatz zur Herstellung von 10L einer Eisen-Zucker / Toltrazuril Dispersion (22,8% m/V aktives Eisen + 5% m/V Toltrazuril) zur oralen Anwendung beim Saugferkel:

| **Einsatzstoff** | **Masse** / **g** |
|---|---|
| Toltrazuril Suspensionskonzentrat (30%) | 1666,67 |
| Natrium Propionat (Konservierungsmittel) | 17,00 |
| Natrium Benzoat (Konservierungsmittel) | 17,00 |
| Propylenglycol | 1000,00 |
| Wasserfreie Zitronensäure | 400,00 |
| Eisen(III)-Zucker Pulver 35,9% m/m | 6350,98 |
| Wasser ad 10 Liter | 4543,73 |
| Viskositätsregulierende/r Hilfsstoff/e | - |
| Gesamtmasse | 13995,38 |

Je 17,00 g der Konservierungsmittel Natrium Propionat und Natrium Benzoat werden in einem separaten Gefäß in 1000,00 g des Lösemittels Propylenglycol eingewogen und unter Rühren gelöst. Der gesamte Wasseranteil wird in ein Edelstahlgefäß vorgelegt (Koruma Disho; Maschinen-Type: DH V 100/45). Je nach Bedarf und gewünschter Viskosität des Endprodukts kann in diese Wassermenge ein viskositätsregulierender Hilfsstoff (ein sogenannter Verdicker) gelöst bzw. eingearbeitet werden. Im diesem Beispiel 3 wird darauf verzichtet. Die Propylenglycol-Vormischung wird zu der Vorlage in das Edelstahlgefäß gegeben und unter Rühren (20-40 min) homogenisiert. Im nächsten Schritt wird die vorher abgewogene Menge von 1666,67g des Toltrazuril-Dispersionkonzentrats 30% zu dem Ansatz gegeben, 30-40 min gerührt und gleichzeitig mit einem Umlaufhomogenisator (Rotor/Stator-System) bei 2500 U/min für 20 Minuten homogenisiert. Es ist vorteilhaft, durch geeignete Kühlsysteme die Temperatur der Mischung auf 20-30°C zu halten. Im nächsten Schritt werden 6350,98g des Eisen(III)-Zucker Pulvers portionsweise zu der Dispersion gegeben. Während der Zugabe muss ständig gerührt und mit dem Umlaufhomogenisator bei 2500 U/min homogenisiert werden. Die genannten Rühr- und Homogenisierzeiten der Suspension können je nach Aussehen der Formulierung verlängert oder verkürzt werden. Die Temperatur wird dabei durch Aktivierung der Kühlung bei 20-30°C gehalten. Nach vollständiger Zugabe des Eisen(III)-Zucker Pulvers wird die Zitronensäure (400,00 g) zu der Mischung gegeben und unter Rühren gelöst. Nach Einarbeitung aller Komponenten wird nochmals 20 Minuten gerührt und mit 2500 U/min nachhomogenisiert. Während dieser Nachrührphase wird die Temperatur der Dispersion durch Kühlung auf Raumtemperatur gehalten. Nach kurzer Zeit wird ein pH Wert von 5 erreicht. Die fertige Dispersion wird über ein 0,1mm Maschensieb aus dem Edelstahlgefäß in geeignete Vorratsbehälter überführt.

| Eisen-Verbindung | Viskosität / mPas | pH nach Herstellung | pH nach Lagerung | Partikelgröße d(v,50) / µm | Partikelgröße d(v,90) / µm | Prozentsatz Partikel < 10µm | Prozentsatz Partikel < 30µm |
|---|---|---|---|---|---|---|---|
| Eisen(III)-Zucker Pulver 35,9% m/m | 1312 | 5,0 | - | 2,1 | 4,3 | 100% | 100% |

### Beispiel 5

### Verwendung von Eisen(III)-Polymaltose Pulver 32,0%ig m/m

Ansatz zur Herstellung von 10L einer Eisen-Zucker / Toltrazuril Dispersion (22,8% m/V aktives Eisen + 5% m/V Toltrazuril) zur oralen Anwendung beim Saugferkel

| **Einsatzstoff** | **Masse / g** |
|---|---|
| Toltrazuril Suspensionskonzentrat (30%) | 1666,67 |
| Natrium Propionat (Konservierungsmittel) | 17,00 |
| Natrium Benzoat (Konservierungsmittel) | 17,00 |
| Propylenglycol | 1000,00 |
| Wasserfreie Zitronensäure | 604,69 |
| Eisen(III)-Polymaltose Pulver 32,0 % m/m | 7125,00 |
| Wasser ad 10 Liter | 3927,21 |
| Viskositätsregulierende/r Hilfsstoff/e | - |
| Gesamtmasse | 14357,57 |

Je 17,00 g der Konservierungsmittel Natrium Propionat und Natrium Benzoat werden in einem separaten Gefäß in 1000,00 g des Lösemittels Propylenglycol eingewogen und unter Rühren gelöst. Der gesamte Wasseranteil wird in ein Edelstahlgefäß vorgelegt (Koruma Disho; Maschinen-Type: DH V 100/45). Je nach Bedarf und gewünschter Viskosität des Endprodukts kann in diese Wassermenge ein viskositätsregulierender Hilfsstoff (ein sogenannter Verdicker) gelöst bzw. eingearbeitet werden. Im diesem Beispiel 4 wird darauf verzichtet. Die Propylenglycol-Vormischung wird zu der Vorlage in das Edelstahlgefäß gegeben und unter Rühren (20-40 min) homogenisiert. Im nächsten Schritt wird die vorher abgewogene Menge von 1666,67g des Toltrazuril-Dispersionkonzentrats 30% zu dem Ansatz gegeben, 30-40 min gerührt und gleichzeitig mit einem Umlaufhomogenisator (Rotor/Stator-System) bei 2500 U/min für 20 Minuten homogenisiert. Es ist vorteilhaft, durch geeignete Kühlsysteme die Temperatur der Mischung auf 20-30°C zu halten. Im nächsten Schritt werden 7125,00 g des Eisen(III)-Polymaltose Pulvers portionsweise zu der Dispersion gegeben. Während der Zugabe muss ständig gerührt und mit dem Umlaufhomogenisator bei 2500 U/min homogenisiert werden. Die Temperatur wird dabei durch Aktivierung der Kühlung bei 20-30°C gehalten. Nach vollständiger Zugabe des Eisen(III)-Polymaltose Pulvers wird die Zitronensäure (604,69g) zu der Mischung gegeben und unter Rühren gelöst. Nach Einarbeitung aller Komponenten wird nochmals 20 Minuten gerührt und gleichzeitig mit 2500 U/min nachhomogenisiert. Die genannten Rühr- und Homogenisierzeiten können je nach Aussehen der Formulierung verlängert oder verkürzt werden. Während der Nachrührphase wird die Temperatur der Dispersion durch Kühlung auf Raumtemperatur gehalten. Die fertige Dispersion wird über ein 0,1mm Maschensieb aus dem Edelstahlgefäß in geeignete Vorratsbehälter überführt. Der pH-Wert steigt nach einigen Tagen bis Wochen Lagerzeit auf werte von 4,8-5,2 an.

| Eisen-Verbindung | Viskosität / mPas | pH nach Herstellung | pH nach Lagerung | Partikelgröße d(v,50) / µm | Partikelgröße d(v,90) / µm | Prozentsatz Partikel < 10µm | Prozentsatz Partikel < 30µm |
|---|---|---|---|---|---|---|---|
| Eisen(III)-Polymaltose Pulver 32.0% m/m | 1226 | 4,4 | 5,0 | 1,9 | 3,3 | 100% | 100 % |

### Beispiel 6

### Verwendung von Eisen(III)-Dextran Pulver 37,9%ig m/m unter Einsatz eines viskositätsregulierenden Hilfsstoffs

Ansatz zur Herstellung von 10L einer Eisen-Zucker / Toltrazuril Dispersion (22,8% m/V aktives Eisen + 5% m/V Toltrazuril) zur oralen Anwendung beim Saugferkel

| **Einsatzstoff** | **Masse / g** |
|---|---|
| Toltrazuril Suspensionskonzentrat (30%) | 1666,67 |
| Natrium Propionat (Konservierungsmittel) | 17,00 |
| Natrium Benzoat (Konservierungsmittel) | 17,00 |
| Propylenglycol | 1000,00 |
| Wasserfreie Zitronensäure | 70,00 |
| Eisen(III)-Dextran Pulver 37,9 % m/m | 6015,83 |
| Bentonite (Veegum) zur Viskositätsregulierung | 20,00 |
| Xanthan Gum zur Viskositätsregulierung | 30,00 |
| Wasser ad 10 Liter | 3290,00 |
| Gesamtmasse | 12126,50 |

Je 17,00 g der Konservierungsmittel Natrium Propionat und Natrium Benzoat werden in einem separaten Edelstahlgefäß in 1000,00 g des Lösemittels Propylenglycol eingewogen und unter Rühren gelöst. Sind die Konservierungsmittel gelöst, werden 30,0 g des Xanthan Gums zugegeben und erneut für etwa 10 Minuten gerührt. Anschließend wird mit einem Umlaufhomogenisator (Rotor/Stator-System; Labor Ultra-Turrax) bei 13.500 U/min etwa 5 Minuten homogenisiert, so dass die Dispersion frei von Klümpchen ist.

Der gesamte Wasseranteil von 3290,0g wird in ein Edelstahlgefäß vorgelegt (Koruma Disho; Maschinen-Type: DH V 100/45). Nachfolgend werden 20g des Bentonits eingestreut. Dieser Ansatz wird jetzt auf 78°C erwärmt und dabei leicht gerührt (50 U/min des Umlaufrührwerks). Die Temperatur sollte etwa 5-10 Minuten auf 78°C gehalten werden, dann unter Rühren und Einschaltung des Kühlsystems auf 35°C abgekühlt werden. 20-40 Minuten wird weitergerührt und unter fortdauernder Kühlung gleichzeitig für 20 Minuten mit dem Umlaufhomogenisator bei 2500 U/min nachhomogenisiert. Die Dispersion des Xanthan Gums in Propylenglycol wird im Anschluss daran unter ständigem Rühren zu der Bentonit/Wasser-Mischung gegeben. Der Ansatz wird dann durch weitere 20-40 Minuten Rühren bei 50 U/min sowie gleichzeitig für 10 Minuten mit dem Umlaufhomogenisators bei 2500 U/min nachhomogenisiert. Auch hier können die genannten Rühr- und Homogenisierzeiten je nach Bedarf und Aussehen der Dispersion verlängert oder verkürzt werden.

Im nächsten Schritt wird die vorher abgewogene Menge von 1666,67g des Toltrazuril-Dispersionkonzentrats 30% zu dem Ansatz gegeben und 30-40 min gerührt und gleichzeitig mit dem Umlaufhomogenisator bei 2500 U/min für 20 Minuten homogenisiert. Es ist vorteihaft, durch effiziente Kühlung die Temperatur der Mischung während der Zugabe auf 20-30°C zu halten. Im nächsten Schritt werden 6015,83g des Eisen(III)-Dextran Pulvers in mehreren Portionen zu der Dispersion gegeben. Während der Zugabe muss ständig gerührt und mit dem Umlaufhomogenisator bei 2500 U/min homogenisiert werden. Die Temperatur wird dabei durch Aktivierung der Kühlung bei 20-30°C gehalten. Nach vollständiger Zugabe des Eisen(III)-Dextran Pulvers wird die Zitronensäure (70,0g) unter Rühren und Homogenisieren zu der Mischung gegeben und gelöst. Der pH stellt sich auf 4,1 - 4,4 ein. Nach Einarbeitung aller Komponenten wird nochmals 20 Minuten gerührt und mit 2500 U/min nachhomogenisiert. Während dieser Nachrührphase wird die Temperatur der Dispersion durch Kühlung auf Raumtemperatur gehalten. Die fertige Dispersion wird über ein 0,1mm Maschensieb aus dem Edelstahlgefäß in geeignete Vorratsbehälter überführt.

| Eisen-Verbindung | Viskosität / mPas | pH nach Herstellung | pH nach Lagerung | Partikelgröße d(v,50) / µm | Partikelgröße d(v,90) / µm | Prozentsatz Partikel < 10µm | Prozentsatz Partikel < 30µm |
|---|---|---|---|---|---|---|---|
| Eisen(III)-Dextran Pulver 37,9% m/m | 1365 | 4,5 | - | 1,7 | 3,5 | 100 % | 100 % |

### Beispiel 7

### Verwendung von Eisene(III)-Dextran Pulver 38,6%ig m/m unter Einsatz eines viskositätsregulierenden Hilfsstoffs

Ansatz zur Herstellung von 1L einer Eisen-Dextran / Toltrazuril Dispersion (20% m/V aktives Eisen + 3% m/V Toltrazuril) zur oralen Anwendung beim Saugferkel

| **Einsatzstoff** | **Masse / g** |
|---|---|
| Toltrazuril Suspensionskonzentrat (30%) | 100,00 |
| Natrium Propionat (Konservierungsmittel) | 1,80 |
| Natrium Benzoat (Konservierungsmittel) | 1,80 |
| Propylenglycol | 100,00 |
| Wasserfreie Zitronensäure | 8,67 |
| Eisen(III)-Dextran Pulver 38,6 % m/m | 518,13 |
| Bentonite (Veegum) zur Viskositätsregulierung | 1,33 |
| Xanthan Gum zur Viskositätsregulierung | 2,00 |
| Wasser ad 1 Liter | 606,42 |
| Gesamtmasse | 1340,15 |

Je 1,80 g der Konservierungsmittel Natrium Propionat und Natrium Benzoat werden in einem separaten Becherglas in 100,00 g des Lösemittels Propylenglycol eingewogen und unter Rühren gelöst. Sind die Konservierungsmittel gelöst, werden 2,00 g des Xanthan Gums zugegeben und erneut für etwa 10 Minuten gerührt, so dass die Dispersion frei von Klümpchen ist.

Etwa 100 g des Wasseranteils werden in einem Becherglas vorgelegt und auf 70-80°C erwärmt. Nachfolgend werden 1,33 g des Bentonits eingestreut und die Temperatur für etwa 5-10 Minuten gehalten. Der entstandene Bentonit-Schleim wird unter Rühren abgekühlt und anschließend der restliche Wasseranteil von 506,42 g zugegeben. Die Bentonit/Wasser-Mischung wird mit Hilfe einer Dissolverscheibe bei 270 U/min gerührt und die Dispersion des Xanthan Gums in Propylenglycol wird unter ständigem Rühren zugegeben. Der Ansatz wird dann für weitere 5-10 Minuten unter Rühren nachhomogenisiert. Auch hier können die genannten Rührzeiten je nach Bedarf und Aussehen der Dispersion verlängert oder verkürzt werden.

Im nächsten Schritt wird die vorher abgewogene Menge von 100,00 g des Toltrazuril-Dispersionkonzentrats 30% zu dem Ansatz gegeben und 30-40 min bei einer Rührgeschwindigkeit von 460 U/min gerührt. Anschließend werden 518,13 g des Eisen(III)-Dextran Pulvers in mehreren Portionen unter ständigem Rühren zu der Dispersion gegeben. Nach vollständiger Zugabe des Eisen(III)-Dextran Pulvers wird die Zitronensäure (8,67 g) unter Rühren zu der Mischung gegeben und gelöst. Der pH stellt sich auf 4,1 - 4,4 ein. Nach Einarbeitung aller Komponenten wird die Suspension für 20 Minuten mit Hilfe eines Rotor-Stator-Homogenisators bei 9.500 U/min nachhomogenisiert. Die fertige Dispersion wird in eine geeignete PE-Flasche überführt.

| Eisen-Verbindung | Viskosität /mPas | pH nach Herstellung | pH nach Lagerung | Partikelgröße d(v,50) / µm | Partikelgröße d(v,90) / µm | Prozentsatz Partikel < 10µm | Prozentsatz Partikel < 30µm |
|---|---|---|---|---|---|---|---|
| Eisen(III)-Dextran Pulver 38,6% m/m | 108 | 4,2 | - | 1,9 | 3,6 | 100 % | 100% |

Die Messergebnisse für die Dispersionen der Beispiele 1-7 zeigen, dass sich erfindungsgemäße Formulierungen mit sehr feinem Feststoffanteil herstellen lassen. Eine unerwünschte Agglomeratbildung wird nicht beobachtet. Zudem befinden sich in den Suspensionen der Beispiele die für die Anwendung bei Saugferkeln notwendigen Inhaltsstoffe 35-44mg Toltrazuril und 200mg aktives Eisen in 0,9ml der Dispersion.

Die Viskosität der Dispersionen der Beispiele 1-7 lässt sich über einen großen Bereich von 10 - 2500 mPas einstellen. Vorteilhaft ist ein weniger viskoser Bereich von 20-1500 mPas, bevorzugt 50-500 mPas, und ganz besonders bevorzugt 20-250 mPas, da er z. B. den Ferkeln geringere Probleme beim Abschlucken bereitet.

### Biologische Beispiele

### Ergebnisse klinischer Versuche mit Formulierungen der Beispiele 2 und 3

Für einen klinischen Versuch standen 30 Zuchtsauen zur Verfügung, die insgesamt 270 Ferkel warfen. Die Aufteilung erfolgte in vier Gruppen, wobei die Würfe jeweils geteilt und je zur Hälfte unterschiedlichen Gruppen zugeteilt wurden. Einer Behandlungsgruppe wurden so, bedingt durch geringfügig unterschiedliche Wurfstärken und -zeitpunkte, zwischen 60 und 75 Ferkel zugeordnet. Es wurden jeweils 0,9 ml der Formulierungen den Ferkeln am Tag 3 nach der Geburt per os verabreicht. Am Tag der Verabreichung, sowie nach 7, 14 und 21 Tagen wurden den Ferkeln Blutproben entnommen. Die Zahl der roten Blutkörperchen (RBC Million Cells / µL), der Hämokritwert (Ht %), der Hämoglobinwert (Hb g/100ml) sowie das Gewicht der Ferkel in kg wurden als Kriterium für die Effizienz der Formulierungen herangezogen. Die Resultate wurden mit der einer Kontrollgruppe verglichen, die ein handelsübliches Eisen(III)-Dextran InjektionsPräparat (Hierrox 200) appliziert am Tag 3 nach der Geburt erhalten hatten. Die Ergebnisse sind in Tabelle 5 dargestellt.

Beide oral applizierten Formulierungen nach Beispiel 2 (aus Eisen(III)-Dextran Pulver hergestellt) und nach Beispiel 3 (aus Eisen(III)-Dextran Lösung hergestellt) bewirkten einen Hämoglobin-Wert von > 9 g/100ml an den Tagen 7, 14 und 21 nach der Geburt und erwiesen sich damit als wirksam zur Vermeidung jeglicher anämischer Mangelzustände. Der Wert von > 10g/100ml 14 und 21 Tage nach der Geburt belegt ferner die sehr gute Bioverfügbarkeit der Formulierungen. Die Kriterien RBC, HT-Wert und Gewichtszunahme ließen zudem keine Nachteile gegenüber dem Injektionspräparat erkennen.

Es kann somit gezeigt werden, dass die einmalige orale Gabe von 200mg aktiven Eisens aus Eisen(III)-Dextran in Verbindung mit Toltrazuril in den erfindungsgemäßen Formulierungen überraschenderweise - entgegen der Lehrmeinung und dem bisherigen Stand der Technik - selbst bei Verabreichung am Tag 3 nach der Geburt eine gute Vorbeugung gegen anämische Mangelerscheinungen bei Saugferkeln ermöglicht.

Eine Analyse der Faeces der Ferkel auf Oocysten ergab einen durchweg negativen Befund. Damit ist nachgewiesen, dass die genannten Formulierungen ebenso effektiv gegen Coccidioseerreger wirksam sind.

Zudem konnten keinerlei negative Begleiterscheinungen wie z.B. Durchfall, wie er bei der oralen Verabreichung hoher Dosen von Eisenverbindungen häufiger beobachtet werden kann, festgestellt werden. Die erfindungsgemäß hergestellten Formulierungen erwiesen sich damit als sehr verträglich.

**Tabelle: Resultate der klinischen Versuche**

| Formulierung nach Beispiel | Tag | Zahl der Ferkel N | RBC [10⁶/ µL] | S.D. | HT [%] | S.D. | Hb [g/100ml] | S.D. | | Tag | Zahl der Ferkel N | Gewicht [kg] | S.D. |
|---|---|---|---|---|---|---|---|---|---|---|---|---|---|
| 2 (oral) | | | | | | | | | | 3 | 70 | 2,192 | 0,051 |
| 3 (oral) | | | | | | | | | | 3 | 74 | 2,116 | 0,050 |
| Injektion i.m. | | | | | | | | | | 3 | 64 | 2,006 | 0,053 |
| 2 (oral) | 7 | 67 | 4,29 | 0,24 | 33,71 | 0,72 | 10,05 | 0,18 | | 7 | 70 | 3,154 | 0,084 |
| 3 (oral) | 7 | 71 | 4,53 | 0,23 | 31,91 | 0,70 | 9,41 | 0,17 | | 7 | 74 | 3,045 | 0,082 |
| Injektion i.m. | 7 | 62 | 4,18 | 0,25 | 33,22 | 0,75 | 9,75 | 0,19 | | 7 | 64 | 2,955 | 0,084 |
| 2 (oral) | 14 | 67 | 5,41 | 0,40 | 39,74 | 0,82 | 11,37 | 0,32 | | 14 | | | |
| 3 (oral) | 14 | 72 | 6,09 | 0,39 | 41,85 | 0,78 | 12,16 | 0,31 | | 14 | | | |
| Injektion i.m. | 14 | 61 | 5,61 | 0,42 | 43,53 | 0,85 | 12,84 | 0,33 | | 14 | | | |
| 2 (oral) | 21 | 64 | 5,48 | 0,11 | 35,66 | 3,33 | 10,78 | 0,36 | | 21 | 68 | 7,061 | 0,146 |
| 3 (oral) | 21 | 72 | 5,69 | 0,10 | 38,71 | 3,10 | 11,61 | 0,34 | | 21 | 73 | 7,173 | 0,141 |
| Injektion i.m. | 21 | 60 | 5,96 | 0,11 | 40,32 | 3,44 | 12,51 | 0,37 | | 21 | 63 | 6,974 | 0,152 |

Die Zahl der roten Blutkörperchen (RBC Million Cells / µL), der Hämokritwert (Ht %), der Hämoglobinwert (Hb g/100ml) sowie das Gewicht der Ferkel in kg wurden als Kriterium für die Effizienz der Formulierungen herangezogen.

## Patentansprüche

1. Formulierung enthaltend Triazinone der Formel (I) oder (II), 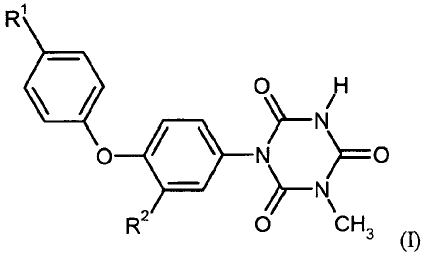 oder 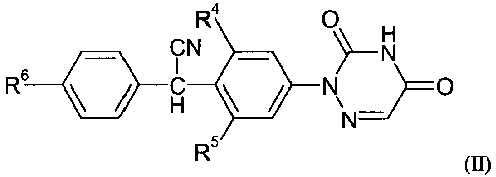 eine polynuklearen Eisen(III)-polysaccharid-Komplexverbindungen und Simethicon.

2. Formulierung gemäß Anspruch 1, wobei der Anteil an Simethicon zwischen 0,01 bis 1 Gew.-% beträgt.

3. Formulierung gemäß einem der vorstehenden Ansprüche, enthaltend 10 bis 90 Gew-% Wasser.
